(19) **Europäisches Patentamt / European Patent Office / Office européen des brevets**

(11) **EP 2 804 963 B1**

(12) **EUROPÄISCHE PATENTSCHRIFT**

(45) Veröffentlichungstag und Bekanntmachung des Hinweises auf die Patenterteilung:
**14.09.2016 Patentblatt 2016/37**

(21) Anmeldenummer: **13700234.1**

(22) Anmeldetag: **15.01.2013**

(51) Int Cl.:
*C22C 38/18* (2006.01)       *C22C 38/38* (2006.01)
*A61L 27/04* (2006.01)       *A61L 31/02* (2006.01)
*A61F 2/82* (2006.01)         *C21D 6/00* (2006.01)
*C22C 38/00* (2006.01)       *C22C 38/02* (2006.01)
*C22C 38/44* (2006.01)       *C22C 38/58* (2006.01)

(86) Internationale Anmeldenummer:
**PCT/EP2013/050652**

(87) Internationale Veröffentlichungsnummer:
**WO 2013/107730 (25.07.2013 Gazette 2013/30)**

(54) **NICKELFREIE EISENLEGIERUNG FÜR STENTS**

NICKEL-FREE IRON ALLOY FOR STENTS

ALLIAGE DE FER SANS NICKEL POUR STENTS

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(30) Priorität: **18.01.2012 EP 12151650**
**09.02.2012 US 201261633324 P**

(43) Veröffentlichungstag der Anmeldung:
**26.11.2014 Patentblatt 2014/48**

(73) Patentinhaber: **MeKo**
**Laserstrahl-Materialbearbeitungen e.K.**
**31157 Sarstedt (DE)**

(72) Erfinder:
• **MEYER-KOBBE, Clemens**
**31157 Sarstedt (DE)**
• **ROSENTRETER, Mark**
**31535 Neustadt (DE)**

(74) Vertreter: **Arth, Hans-Lothar**
**ABK Patent Attorneys**
**Jasminweg 9**
**14052 Berlin (DE)**

(56) Entgegenhaltungen:
**EP-A1- 0 640 695       EP-A2- 1 087 029**
**DE-C1- 19 513 407     US-B1- 6 267 921**

**Beschreibung**

Die vorliegende Erfindung betrifft eine weitgehend nickelfreie Eisenlegierung bzw. einen nickelfreien Edelstahl, der insbesondere zur Herstellung von Stents geeignet ist und daraus hergestellte Stents.

Die Implantation von Gefäßstützen wie beispielsweise Stents ist heutzutage ein gängiger chirurgischer Eingriff zur Behandlung von Stenosen. Sie werden üblicherweise aus Metalllegierungen wie nicht-rostendem Edelstahl oder Nitinol hergestellt. Derartige Metallstents sind in großer Zahl bekannt und haben sich in der Praxis bewährt. Auf Grund ihrer Metallstruktur und Tragkraft sollen derartige Metallstents gewährleisten, dass die Gefäße nach Implantation offen bleiben und der Blutfluss durch die Gefäße dauerhaft gewährleistet wird. Zum anderen dienen Stents in der Krebsbehandlung dazu, durch bösartige Tumoren verursachte Verengungen von Atemwegen (Luftröhre), Gallenwegen oder der Speiseröhre nach einer Aufdehnung offenzuhalten.

Ein Stent ist ein kleines Gittergerüst in Form eines Röhrchens. Es wird über einen Katheter bis zu der Stelle vorgeschoben, wo ein Blutgefäß durch Arteriosklerose verengt ist. Dort wird das Gittergerüst mit Hilfe eines Ballons von innen an die Gefäßwand gedrückt. Die Wand wird gedehnt und die Engstelle geweitet. Der Stent soll dabei sicherstellen, dass sich die Arterie nicht mehr zusammenziehen kann, ein Risiko, dass auf Grund der elastischen Rückstellkraft einer Gefäßwand besteht. Mit der Zeit wachsen die Zellen der Gefäßwand um den Stent herum, so dass er eine Stütze innerhalb der Arterie ist. Dies kann jedoch durch auftretende Entzündungsreaktionen verzögert werden.

Der menschliche Körper kann beim Kontakt mit bestimmten Stoffen eine Überempfindlichkeitsreaktion, insbesondere Allergien ausbilden. Dabei handelt es sich um eine überschießende Abwehrreaktion des Immunsystems auf bestimmte Umweltstoffe (Allergene), die oft mit entzündlichen Prozessen einhergehen. Somit besteht auch die Gefahr einer Störung der Wundheilung, was mit einem erhöhten Thromboserisiko einhergeht. Die expositionsbedingten Symptome einer Allergie können mild bis schwerwiegend und in einigen Fällen sogar akut lebensbedrohlich sein. Nickel ist heute eines der häufigsten Kontaktallergene.

Rostfreie Stähle aus dem Stand der Technik (z.B. 18/10 CrNi, 316L) enthalten vermehrt Nickel. Daher können diese bei Kontakt mit dem menschlichen Körper eine Nickelallergie auslösen. Es gibt daher in verschiedenen europäischen Ländern gesetzgeberische Maßnahmen, die den Gebrauch nickelhaltiger Werkstoffe am und im menschlichen Körper verbieten bzw. einschränken. Die europäische Richtlinie 94/27/EG setzt z.B. einen Grenzwert für die Freisetzung von Nickel für Produkte, die unmittelbar und länger mit der Haut in Berührung kommen.

Kobalt ist auch ein weit verbreitetes Kontaktallergen, das expositionsbedingt eine Überempfindlichkeitsreaktion oder Allergie ähnlich wie Nickel auslösen kann. Kobalt ist der Hauptbestandteil von Kobalt-Chrom Legierungen, die aufgrund ihrer hervorragenden mechanischen Eigenschaften für die Herstellung von Stents genutzt werden. Diese können jedoch bei Kontakt mit dem menschlichen Körper eine Allergie auslösen, häufig tritt diese als Kreuzreaktion mit einer Nickelallergie auf. Man geht davon aus, dass ein Drittel aller Nickelallergiker auch Reaktionen auf Kobalt zeigen.

Das Patent US 6,508,832 offenbart Stents aus dem nickelfreiem Edelstahl Bio Dur® 108 von Carpenter Technologies, USA. In Versuchen der Erfinder dieser Anmeldung war die Dehngrenze ($R_{p0,2}$ ~ 800 MPa) dieser Legierung jedoch so hoch, dass diese Legierung in Kombination mit einem relativ geringen E-Modul (~ 195GPa) eine große elastische Verformbarkeit aufweist. Wird der Stent aus dieser Legierung standardmäßig auf einen Katheterballon gekrimpt federt dieser elastisch zurück (Spring Back), so dass der Stent nicht fest genug auf dem Ballon sitzt und während der Implantation vom Ballon rutschen kann.

Die Patentanmeldung EP 640 695 A1 offenbart eine nickelfreie, austenitische Legierung zur Herstellung von Produkten mit Hautkontakt. Die Zusammensetzung der Legierung gemäß EP 640 695 A1 enthält die gleichen Elemente, wie die vorliegende Erfindung, unterscheidet sich aber insbesondere im Stickstoff und Kohlenstoffgehalt. Implantate und insbesondere Stents aus dieser Legierung werden jedoch nicht beschrieben.

Die Patentanmeldung EP 1 087 029 A2 betrifft Nickel-freie Stahllegierungen für medizinische Implantate, jedoch nicht gezielt Stents. Die Zusammensetzung der Legierung aus EP 1 087 029 A2 unterscheidet sich von der Zusammensetzung der Legierung der vorliegenden Erfindung vor allem durch die geringere Masse an Molybdän, welche sich als wenig geeignet für die Herstellung von Stents erwiesen hat. Ferner offenbart die europäische Patentanmeldung EP 0 875 591 B1 die Verwendung einer austenitischen Stahllegierung für die Herstellung von am oder im Körper getragenen Gegenständen. Bei einem Stickstoffgehalt von mehr als 0,55 % muss der Kohlenstoffgehalt dabei > 0,3 % sein. Dies hat sich aber für die Herstellung von Stents als nachteilig erwiesen.

Das deutsche Patent DE 195 13 407 C1 beschreibt die Verwendung einer austenitischen Stahllegierung für die Herstellung von am oder im Körper getragenen Gegenständen. Stents aus dieser Legierung werden nicht beschrieben. Es handelt sich bei der vorliegenden Legierung um eine gezielte Auswahl der Legierungselemente, sowie deren verwendeter Mengen die zu einer für Stents optimal geeigneten Zusammensetzung führen.

Aufgabe der vorliegenden Erfindung ist es, eine weitgehend nickelfreie Eisenlegierung bereitzustellen, welche sich insbesondere zur Herstellung von Stents eignet.

Diese Aufgabe wird erfindungsgemäß durch die technische Lehre der unabhängigen Ansprüche gelöst. Weitere vorteilhafte Ausgestaltungen der Erfindung ergeben sich aus den abhängigen Ansprüchen, der Beschreibung sowie den Beispielen.

Es hat sich überraschend gezeigt, dass die erfindungsgemäßen, nickelfreien Stahllegierungen sich durch ein vorteilhaftes Korrosionsverhalten, gewünschte Festigkeit und weiteren für die Herstellung von Stents geeigneten mechanischen Eigenschaften auszeichnen. Zudem können diese Legierungen auf Grund der unkritischen Nickelkonzentration keine Nickelallergien auslösen.

Die vorliegende Erfindung betrifft daher unter anderem eine Stahllegierung, vorzugsweise eine austenitische Stahllegierung, die bezogen auf das Gesamtgewicht der Legierung (angegeben in Gew.-%) folgende Bestandteile enthält:

| | | |
|---|---|---|
| 14,00 Gew.-% - | 16,50 Gew.-% | Chrom |
| 10,00 Gew.-% - | 12,00 Gew.-% | Mangan |
| 3,00 Gew.-% - | 4,00 Gew.-% | Molybdän |
| 0,55 Gew.-% - | 0,70 Gew.-% | Stickstoff |
| 0,10 Gew.-% - | 0,20 Gew.-% | Kohlenstoff |

der Rest bis auf 100 Gew.-% ist Eisen.

Diese Legierung kann ferner noch Verunreinigungen enthalten. Sind neben Chrom, Mangan, Molybdän, Stickstoff und Kohlenstoff noch andere Bestandteile anwesend, so handelt es sich dabei um Verunreinigungen wie beispielsweise andere Metalle, Metallsalze, Nichtmetalle, Schwefel, Sauerstoff, Silizium und/oder Wasserstoff. Die in der Legierung vorhandenen Verunreinigungen sind herstellungsbedingte Verunreinigungen.

Die vorliegende Erfindung stellt eine im wesentlichen nickelfreie Eisenlegierung bereit, welche sich insbesondere zur Herstellung von Stents eignet und den im Stand der Technik bekannten Eisenlegierungen überlegen ist, weil die Einflüsse der einzelnen Legierungsbestandteile an sich als auch in Relation zu den anderen Bestandteilen eingehend untersucht und für jeden Legierungsbestandteil optimale Bereiche und Grenzwerte ermittelt wurden, welche alle zusammengenommen der Legierung überlegene Eigenschaften verleihen.

So ist die Menge an Mangan auf einen kleinen aber vorteilhaften Bereich beschränkt worden, welcher nicht dem in EP 640 695 A1 bevorzugten entspricht. EP 640 695 A1 offenbart zudem Legierungen mit einem geringen Anteil an Molybdän, welcher sich auf Grund der Korrosionsbeständigkeit im Hinblick auf die erfindungsgemäße Legierung als nicht geeignet erwiesen hat. Hinsichtlich der Dehngrenze wird ein geringer Stickstoffgehalt bevorzugt. Im Rahmen der vorliegenden Erfindung wurde zugleich gefunden, dass ein höherer Kohlenstoffgehalt notwendig ist, um die mechanischen Eigenschaften einer Stickstoff reduzierten, nickelfreien Stahllegierung zu erhalten, die für Stents erforderlich sind. Diese Optimierung einer nickelfreien Stahllegierung als Werkstoff für Stents ist keiner der zuvor beschriebenen Patentschriften zu entnehmen. Insbesondere sind die in den Beispielen aus DE 195 13 407 C1 verwendeten Mengen an Stickstoff und Kohlenstoff in einem Bereich, der sich, wie in dieser Anmeldung gezeigt, nachteilig auf die mechanischen Eigenschaften der Legierung auswirkt.

Das europäische Patent EP 1 025 273 B1 offenbart eine nickelfreie, austenitische Legierung zur Herstellung von Produkten mit Körperkontakt. Die Legierungen aus EP 1 025 273 B1 unterscheiden sich von der vorliegenden Erfindung vor allem in der Menge an Mangan, die wesentlich höher liegt (> 15 %). Wie in den Beispielen dieser Anmeldung gezeigt, weisen jedoch nickelfreie Stahllegierungen mit einem höheren Mangangehalt nicht die für Stents gewünschten mechanischen Eigenschaften auf.

Die Patentanmeldung EP 1 579 886 A1 beschreibt medizinische Vorrichtungen, auch Stents, aus einer nickelfreien Eisenlegierung. Die Anmelder von EP 1 579 886 A1 untersuchten vorwiegend den Einfluss von Stickstoff in einer nickelfreien Legierung und bevorzugen einen Mindestgehalt von 0,8 Gew-%. Dies wurde getestet, indem die Festigkeit einer bestimmten Zusammensetzung mit und ohne Stickstoff überprüft wurde. Der Gegenstand dieser Anmeldung beruht jedoch darauf eine optimierte Zusammensetzung durch Abstimmung der Mengen aller Bestandteile zueinander zu entwickeln. Dies führte zu besser geeigneten, engeren Teilbereichen der größeren Bereiche aus EP 1 579 886 A1, die zudem vor allem im Mangan- und Stickstoffgehalt von den im Stand der Technik bevorzugten Bereichen abweichen. So war in der in EP 1 579 886 A1 getesteten Legierung gar kein Mangan enthalten und der Stickstoffgehalt höher.

Die Autoren der wissenschaftlichen Publikation: Chen et al., Computational Materials Science, 2009; 572 - 578 untersuchten die Racheting- und Ermüdungseigenschaften einer X13CrMnMoN18-14-3 Legierung. Das Ergebnis der vorliegenden Erfindung zeigt jedoch, dass es für die mechanischen Eigenschaften von Vorteil ist, wenn die Zusammensetzung einer Legierung diesbezüglich verändert wird. Sowohl der Chrom- als auch der Mangananteil und insbesondere der Stickstoffgehalt sollten niedriger sein, um eine, für die Anwendung als Werkstoff für Stents, optimale Dehngrenze zu erzeugen

Die großen Rückstellkräfte der Gefäße nach einer Aufdehnung und Entzündungsreaktionen sind wesentliche Gründe für Restenosen. Daher müssen insbesondere vaskuläre Gefäßstützen bzw. Stents aus einem Material bestehen, welches gut vom Körper vertragen wird, d.h. keine Allergien oder Unverträglichkeiten auslöst, aber auch eine ausreichend hohe Rückhaltekraft und Stabilität aufweist, um einen erneuten Verschluss des Gefäßes zu verhindern.

Ein einmal eingesetzter Stent muss seine Größe und Form beibehalten, trotz der unterschiedlichen Kräfte, die auf ihn einwirken, wie z.B. die pulsierende Belastung durch das schlagende Herz. Darüber hinaus muss der Stent genügend Flexibilität besitzen, um auf einen Ballon gekrimpt und später im Gefäß expandiert werden zu können.

Aus diesem Grund besteht das Bedürfnis einen geeigneten, nickelfreien Werkstoff für Stents zu entwickeln. Aufgabe der vorliegenden Erfindung ist es, einen besonders gut geeigneten Werkstoff und eine daraus hergestellte Gefäßstütze bereitzustellen.

Die erfindungsgemäße Legierung kann herstellungsbedingte Verunreinigungen wie z.B. weitere Metalle, Halbmetalle, Metallsalze und/oder Nichtmetalle in geringen Mengen bis zu maximal 2,0 Gew.-% aller weiterer Komponenten zusammen enthalten. Bei den weiteren Bestandteilen handelt es sich vorzugsweise um Nickel, Titan, Niob, Silizium, Schwefel und Phosphor, wobei die Höchstgrenze für Nickel bei 0,05 Gew.-% in der Legierung liegt. Titan und Niob können jeweils in einer Menge von bis zu 0,07 Gew.-%, bevorzugt 0,05 Gew.-% und besonders bevorzugt 0,02 Gew.-% in der Legierung enthalten sein. Silizium kann in einer Menge von bis zu 0,1 Gew.-% und bevorzugt 0,50 Gew.-% und Phosphor in einer Menge von bis zu 0,05 Gew.-% in der Legierung zugegen sein. Die Gesamtmenge an Verunreinigungen neben Chrom, Mangan, Molybdän, Stickstoff, Kohlenstoff und Eisen sollte insgesamt 2,0 Gew.-%, vorzugsweise 1,6 Gew.-%, weiter bevorzugt 1,4 Gew.-%, noch weiter bevorzugt 1,2 Gew.-%, noch weiter bevorzugt 1,1 Gew.-% und am meisten bevorzugt 1,0 Gew.-% nicht übersteigen.

Es versteht sich von selbst, dass alle Bestandteile einer Legierung zusammen 100 Gew.-% ergeben müssen. Enthält obige Legierung somit 16,5 Gew.-% Chrom (Cr) und 12,0 Gew.-% Mangan (Mn) sowie 4,0 Gew.-% Molybdän (Mo), 0,70 Gew.-Stickstoff (N) und 0,20 Gew.-% Kohlenstoff (C) so kann der Eisenanteil (Fe) nicht über 66,60 Gew.-% liegen.

Sofern nicht explizit aufgeführt, können die hierin offenbarten Legierungen herstellungsbedingte Verunreinigungen enthalten, welche im Bereich von der Nachweisgrenze oder im Bereich von 1 ppm bis zu 2,0 Gew.-%, bevorzugt bis zu 1,8 Gew.-%, weiter bevorzugt bis zu 1,5 Gew.-% und besonders bevorzugt bis zu 1,2 Gew.-% liegen. Silizium als Hauptbestandteil der Verunreinigungen kann dabei bereits bis 1,0 Gew.-%, bevorzugt bis zu 0,9 Gew.-% ausmachen. Es ist daher besonders bevorzugt, wenn die herstellungsbedingetn Verunreinigungen außer Silizium insgesamt weniger als 1,0 Gew.-%, bevorzugt weniger als 0,8 Gew.-%, weiter bevorzugt 0,5 Gew.-%, weiter bevorzugt 0,2 Gew.-%, weiter bevorzugt 0,1 Gew.-%, weiter bevorzugt 0,05 Gew.-%, weiter bevorzugt weniger als 0,01 Gew.-% und insbesondere bevorzugt weniger als 500 ppm betragen. Die vorgenannten Prozentzahlen beziehen sich auf die Summe aller Verunreinigungen außer Silizium und nicht auf die einzelne Verunreinigung. Diese Verunreinigungen (einschließlich Si) können auch dann in einer Menge von 1 ppm bis hin zu 2,0 Gew.-% oder 1,8 Gew.-% oder 1,5 Gew.-% oder 1,2 Gew.-% in der Legierung vorhanden sein, wenn sie nicht explizit als Legierungsbestandteil aufgeführt sind und werden im Fall der Nichtnennung dem Gewichtsanteil des Bestandteils der Legierung zugerechnet, durch den sie in die Legierung gelangt sind. Bevorzugt ist jedoch, wenn die Verunreinigungen außer Silizium jeweils, d.h. auf das einzelne Element bezogen, eine Menge von 0,1 Gew.-%, weiter bevorzugt 0,05 Gew.-%, weiter bevorzugt weniger 0,01 Gew.-%, bevorzugt 500 ppm, weiter bevorzugt 300 ppm und insbesondere bevorzugt 150 ppm nicht überschreiten. Silizium kann ein Hauptbestandteil der Verunreinigungen sein und bis zu 1,0 Gew.-% und bevorzugt bis zu 0,8 Gew.-% in der Legierung vorhanden sein.

Die Erfindung umfasst ferner Stahllegierungen, die bezogen auf das Gesamtgewicht der Legierung aus folgenden Bestandteilen bestehen:

| | | |
|---|---|---|
| 14,0 Gew.-% - | 16,5 Gew.-% | Chrom |
| 10,0 Gew.-% - | 12,0 Gew.-% | Mangan |
| 3,0 Gew.-% - | 4,0 Gew.-% | Molybdän |
| 0,55 Gew.-% - | 0,70 Gew.-% | Stickstoff |
| 0,10 Gew.-% - | 0,20 Gew.-% | Kohlenstoff |

1 ppm- 2,0 Gew.-% Verunreinigungen in Form von anderen Metallen (d.h. andere als Chrom, Mangan, Molybdän und Eisen) in jeweils einer Maximalmenge bis zu 0,075 Gew.-%, und Nichtmetalle aus der Gruppe S, Si, P in einer maximalen Gesamtmenge von 1,2 Gew.-%, der Rest bis auf 100 Gew.-% ist Eisen.

Die Bezeichnung "Nichtmetalle aus der Gruppe S, Si, P in einer maximalen Gesamtmenge von 1,2 Gew.-%" bedeutet, dass der Anteil an Schwefel, Phosphor und Silizium zusammen die maximale Menge von 1,2 Gew.-% nicht überschreitet, wobei es bevorzugt ist, dass Silizium bis zu 1,0 Gew% ausmacht und Schwefel und Phosphor zusammen nicht mehr als 0,2 Gew% beitragen.

Es ist bevorzugt, wenn es sich bei den erfindungsgemäßen Legierungen um austenitische Stahllegierungen handelt. Eine bevorzugte Zusammensetzung einer erfindungsgemäßen Stahllegierung enthält neben Chrom, Mangan, Molybdän, Stickstoff, Kohlenstoff des Weiteren 0,00 Gew.-%- 0,05 Gew.-% Nickel und/oder 0,00 Gew.-% - 1,00 Gew.-

% Silizium.

Eine bevorzugte Zusammensetzung einer erfindungsgemäßen Stahllegierung besteht bezogen auf das Gesamtgewicht der Legierung aus den folgenden Bestandteilen:

| | | |
|---|---|---|
| 14,0 Gew.-% - | 16,5 Gew.-% | Chrom |
| 10,0 Gew.-% - | 12,0 Gew.-% | Mangan |
| 3,0 Gew.-% - | 4,0 Gew.-% | Molybdän |
| 0,55 Gew.-% - | 0,70 Gew.-% | Stickstoff |
| 0,10 Gew.-% - | 0,20 Gew.-% | Kohlenstoff |
| 0,00 Gew.-% - | 0,05 Gew.-% | Nickel |
| 0,00 Gew.-% - | 1,00 Gew.-% | Silizium (weiter bevorzugt bis 0,5 Gew.-%) |
| 0,00 Gew.-% - | 0,1 Gew.-% | Phosphor und Schwefel |

und der Rest bis auf 100 Gew.-% ist Eisen und unvermeidbare Verunreinigungen.

Bei der vorgenannten Legierung sind die anderen Metalle (d.h. andere als Chrom, Mangan, Molybdän, Nickel und Eisen) vorzugsweise jeweils in einer Maximalmenge von 0,05 Gew.-% und die anderen Nichtmetalle (d.h. andere als Stickstoff, Kohlenstoff und Silizium) jeweils in einer Maximalmenge von 0,05 Gew.-% in der vorgenannten Legierung enthalten.

Die erfindungsgemäße Stahllegierung enthält 0,10 Gew.-% - 0,20 Gew.-% Kohlenstoff. Es ist bevorzugt, wenn eine erfindungsgemäße Stahllegierung bezogen auf das Gesamtgewicht der Legierung 0,12 Gew.-% - 0,20 Gew.-% und weiter bevorzugt 0,14 Gew.-% - 0,19 Gew.-% und noch mehr bevorzugt 0,16 Gew.-%-0,18 Gew.-% Kohlenstoff enthält.

Es ist zudem bevorzugt, wenn die Masse an C und N zusammen mehr als 0,70 Gew.-% beträgt, weiter bevorzugt mehr als 0,75 Gew.-%, noch weiter bevorzugt mehr als 0,80 Gew.-% beträgt und noch weiter bevorzugt zwischen 0,80 Gew.-% und 0,90 Gew.-% und insbesondere bevorzugt zwischen 0,83 Gew.-% und 0,87 Gew.-% liegt.

Chrom fördert bei hohen Gehalten die Entstehung von Deltaferrit und Sigmaphasen und verkleinert den austenitischen Bereich, weshalb der Chromgehalt begrenzt werden muss. Ein Chromgehalt von 17,00 Gew.-% und mehr hat sich jedoch als ungeeignet für die erfindungsgemäße Legierung erwiesen. Andererseits erhöht Chrom die Korrosionsbeständigkeit, die Stickstofflöslichkeit und verbessert die Polierbarkeit, so dass Chrom dennoch einen wesentlichen Bestandteil der Legierung darstellt.

Es ist somit bevorzugt, wenn die erfindungsgemäße Legierung 14,0 - 16,5 Gew.-%, bevorzugt 14,5 - 16,3 Gew.-%, weiter bevorzugt 14,8 - 16,2 Gew.-%, weiter bevorzugt 15,0 - 16,1 Gew.-%, noch weiter bevorzugt 15,2 - 16,0 Gew.-% Chrom aufweist.

Mangan bildet bei hohen Gehalten intermetallische Phasen aus, die die Korrosionsbeständigkeit verringern und zur Versprödung des Materials führen. Des Weiteren führt ein hoher Mangangehalt aufgrund der hohen chemischen Aktivität zu einer schlechten Polierbarkeit. Im Stand der Technik sind Eisenlegierungen mit einem Mangangehalt von weit über 18 Gew.-% bekannt. Diese Legierungen wurden zu Vergleichszwecken auch getestet und haben sich auch wegen der schlechten Polierbarkeit als nicht einsetzbar erwiesen.

Der so genannte MARC-Wert (Measure of Alloying for Resistance to Corrosion) ist der neuste Ansatz die chemische Beständigkeit zu berechnen. Er basiert auf dem PRE-Wert (Pitting Resistance Equivalent) und ist um die Elemente Kohlenstoff, Mangan und Nickel erweitert.

$$\text{MARC} = [\%Cr] + 3{,}3 \times [\%Mo] + 20 \times [\%C] + 20 \times [\%N] - 0{,}5 \times [\%Mn] - 0{,}25 [\%Ni]$$

Anhand der Formel für den MARC-Wert wird ersichtlich, dass Mangan die Korrosionsbeständigkeit verringert.

Folgende Formel stellt unter anderem den Einfluss von Mangan auf die Dehngrenze dar:

$$\text{Dehngrenze (MPa)} = 251 + 33 \times \text{Mn (m\%)} + 313 \times [\text{N} + \text{C (m\%)}]$$

Die Dehngrenze wird durch die Zugabe von 1 % Mangan um 33 MPa erhöht. Die Verringerung der Korrosionsbeständigkeit und die Erhöhung der Dehngrenze durch die Zugabe von Mangan legen einen geringen Mangangehalt nahe. Andererseits erhöht Mangan die Stickstofflöslichkeit und vergrößert das Austenitgebiet. Dies spricht für einen hohen Mangangehalt.

Bevorzugt liegt die Masse an Mangan daher im Bereich von 10,0 - 12,0 Gew.-%, weiter bevorzugt 10,2 - 11,9 Gew.-%, weiter bevorzugt 10,5 - 11,9 Gew.-%, noch weiter bevorzugt 10,8 - 11,8 Gew.-%, weiter bevorzugt 10,3 - 11,6 Gew.-%, und insbesondere bevorzugt von 11,0 - 11,7 Gew.-%.

Es ist zudem bevorzugt, wenn die erfindungsgemäße Legierung in Anteilen von 3,0 - 4,0 Gew.-%, weiter bevorzugt 3,1 - 3,8 Gew.-% und insbesondere bevorzugt von 3,2 - 3,7 Gew.-% Molybdän enthält.

Molybdän erhöht die Beständigkeit gegen Lochkorrosion in reduzierenden Umgebungen und ist daher als Bestandteil der Legierung ausgewählt worden. Molybdän fördert die Entstehung von Alphaphasen und Sigmaphasen und verschlechtert aufgrund seiner hohen Passivität das Polierergebnis. Des Weiteren ist Molybdän ein starker Ferritbildner. Durch den starken Umformvorgang beim Crimpen (Aufsetzen des Stents auf einen Katheterballon) und Dilatieren werden auch austenitische Werkstoffe zu einem Teil ferritisch. Daher sollte der Werkstoff, durch Verwendung von Austenit fördernden Legierungselementen und Meidung von Ferrit fördernden Legierungselementen, möglichst weit vom Umwandlungspunkt zum ferritischen Werkstoff entfernt liegen. Molybdän ist ein starker Karbidbildner. Die Entstehung von Karbiden ist abhängig vom Kohlenstoffgehalt, vom Gehalt des Karbidbildners und von der Wärmebehandlung in Abhängigkeit des Zustandes des Werkstoffs, wie z.B. der Versetzungsdichte, vor der Wärmebehandlung. Um die gewünschten Bruchdehnungen zu erreichen ist eine starke Veränderung der Gefügestruktur notwendig, was zu einer verstärkten Karbidbildungsneigung führt. Karbide in einer Legierung sind in der Verwendung als Stentmaterial von Nachteil, da Materialinhomogenitäten Rissbildung fördern, zu unpolierten Stellen auf dem Stent und zu einer lokalen Verarmung von Kohlenstoff führen und damit die Korrosionsbeständigkeit und die Festigkeiten verringern. Insofern sollte der Molybdängehalt in den erfindungsgemäßen Legierungen auf 4,0 Gew.-% limitiert werden.

Bei Erhöhung des Stickstoffgehalts steigt die Wahrscheinlichkeit, dass Nitride wie z.B. Chromnitride entstehen. Dies verringert durch die umliegende Chrom- und Stickstoffverarmung die Korrosionsbeständigkeit. Daher sollte im Zusammenhang mit dieser Erfindung eine obere Grenze des Stickstoffgehalts in einer erfindungsgemäßen Stahllegierung ermittelt werden (s. Beispiel 7). Da die Nitridentstehung auch von den Wärmebehandlungsparametern abhängt, muss der Stickstoffgehalt in Abhängigkeit der angewendeten Wärmebehandlung gewählt werden. Stickstoff erhöht die Festigkeit der Legierung. In der Anwendung als Stent, vor allem als vaskuläre Gefäßstütze, ist eine geringe elastische Dehnung gefordert, da der Stent auf einen Ballon gekrimpt wird. Federt das Implantat nach dem Krimpen stark zurück (Spring Back), kann es während der Implantation vom Ballon rutschen. Um beim gegebenen Elastizitätsmodul eine geringe elastische Verformung zu erreichen, muss folglich eine niedrige Dehngrenze ($R_{p0,2}$), bevorzugt kleiner 600 MPa, erzeugt werden. Die Dehngrenze lässt sich in bestimmten Bereichen durch einen geeigneten Wärmebehandlungsprozess und andererseits durch einen niedrigen Stickstoffgehalt einstellen.

Atomar gelöster Stickstoff erhöht die chemische Beständigkeit, so dass eine ausreichende chemische Beständigkeit einer Stahllegierung ohne den Zusatz von Nickel erst möglich wird. Daher ist ein Mindestgehalt von Stickstoff erforderlich. Des Weiteren ist Stickstoff ein starker Austenitbildner, so dass ein Mindestgehalt zur Sicherstellung eines austenitischen Gefüges der Stahllegierung notwendig ist.

Mit höherem Stickstoffgehalt steigt die Dehngrenze von Stahllegierungen. Da die Dehngrenze für die Anwendung als Stent unter 600 MPa liegen muss, ist hinsichtlich der Dehngrenze ein möglichst geringer Stickstoffgehalt gefordert.

Es ist somit bevorzugt, wenn die erfindungsgemäße Legierung 0,50 - 0,70 Gew.-%, bevorzugt 0,55 - 0,70 Gew.-%, bevorzugt 0,58 - 0,69 Gew.-%, weiter bevorzugt 0,60 - 0,68 Gew.-% und noch weiter bevorzugt 0,62 - 0,67 Gew.-%, noch weiter bevorzugt 0,55 - 0,61 Gew.-% und noch besonders bevorzugt 0,56 - 0,59 Gew.-% Stickstoff aufweist.

Durch einen geeigneten Wärmebehandlungsprozess nimmt Kohlenstoff wie Stickstoff Zwischengitterplätze ein und erhöht somit die Festigkeit und vergrößert das Austenitgebiet in einer Stahllegierung. Der Festigkeitsanstieg durch Kohlenstoff ist jedoch geringer als der von Stickstoff. Typischerweise wird der Kohlenstoffgehalt in austenitischen Stählen dennoch stark begrenzt (z.B. auf < 0,06 Gew.-% oder sogar < 0,03 Gew.-%) um die Entstehung von Karbiden wie z.B. Chromkarbid zu vermeiden, da solche Ausscheidungen zu einer Chromverarmung im umliegenden Material und somit zu einer verringerten Korrosionsbeständigkeit führen.

Um den nur sehr schlecht vermeidbaren geringen Anteil an Kohlenstoff am Eingehen einer Verbindung mit Chrom zu hindern wird der Kohlenstoff im Stand der Technik an andere Elemente gebunden, die dazu in geringem Umfang der Legierung zugefügt werden. Elemente die aufgrund Ihrer hohen Affinität zu Kohlenstoff der Legierung typischerweise zugeführt werden sind Titan, Niob und Vanadium. Bekannte austenitische Legierungen weisen somit keinen atomar gelösten Kohlenstoff auf.

Im Zusammenhang mit der erfindungsgemäßen Legierungszusammensetzung kann die Chromkarbidentstehung durch eine geeignete Temperaturführung, die so nur an sehr dünnwandigen Bauteilen durchgeführt werden kann, vermieden werden.

Im Herstellungsprozess einer Legierung kann Kohlenstoff verstärkt Bindungen mit Wolfram eingehen und verringert somit den Anteil an freiem, atomarem Kohlenstoff. Die so entstehenden Karbide sind mit der einer Wärmebehandlung nicht aufzulösen. Daher sollten die erfindungsgemäßen Legierungen möglichst frei von Wolfram sein, bzw. der Wolframgehalt einer erfindungsgemäßen Legierung auf ≤ 0,05 Gew.-% und bevorzugt ≤ 0,02 Gew.-% insbesondere bevorzugt 500 ppm, weiter bevorzugt 300 ppm und insbesondere bevorzugt 150 ppm begrenzt werden.

Die erfindungsgemäße Stahllegierung weist einen Kohlenstoffgehalt von 0,10 Gew.-% bis 0,20 Gew.-% auf. Der Gehalt an Titan, Niob und Vanadium wird bevorzugt auf jeweils maximal 0,02 Gew.-% limitiert um eine Verbindung des Kohlenstoffs mit diesen Elementen zu verhindern. Somit wird sichergestellt, dass der Kohlenstoff, zumindest in wesentlichen Teilen, in atomarer Form vorliegt. In den erfindungsgemäßen Legierungen liegt der Kohlenstoff vorzugsweise zu mindestens 70 Gew.-% in freien, d.h. atomarer Form vor und nicht als Karbid gebunden und weiter bevorzugt zu mindesten 80 Gew.-% und noch weiter bevorzugt zu mindestens 90 Gew.-% in freier oder atomarer Form vor.

Die vorliegende Gitterstruktur kann mittels Röntgenstrukturanalyse bestimmt werden. Dazu werden Röntgenstrahlen am Kristallgitter gebeugt, so dass Interferenzmuster entstehen. Aus diesen Interferenzmustern kann auf die Atomabstände im Kristallgitter geschlossen werden. Durch atomar gelösten Kohlenstoff und Stickstoff werden die Atomabstände beeinflusst, durch inkohärente Ausscheidungen von gebundenem Kohlenstoff oder Stickstoff nicht. Somit kann, bei bekanntem Gesamtgehalt von Kohlenstoff und Stickstoff und durch Bestimmung des atomar gelösten Kohlenstoffs und Stickstoffs durch die Röntgenstrukturanalyse, das Verhältnis von atomar gelöstem Kohlenstoff und Stickstoff und gebundenem Kohlenstoff und Stickstoff bestimmt werden.

Vollständig gelöster Kohlenstoff hat bei Anwesenheit von vollständig gelöstem Stickstoff andere positive Eigenschaften als bei Abwesenheit von Stickstoff, so dass durch die überlagerte Wirkung der beiden Elemente Vorteile für die Legierung erzeugt werden. Dieser positive Effekt gilt für die hier untersuchten Chrom-ManganStähle und ist unter Umständen nicht auf andere Legierungen übertragbar. Des Weiteren wurde festgestellt, dass ein bestimmtes Verhältnis von Stickstoff zu Kohlenstoff die positiven Eigenschaften verstärkt. Um sowohl Kohlenstoff als auch Stickstoff vollständig in einer Legierung zu lösen muss der Chrom- und Mangangehalt entsprechend eingestellt werden und bevorzugt eine an die gegebenen Bedingungen wie Wandstärke und vorliegender Versetzungsdichte angepasste Wärmebehandlung durchgeführt werden.

Kohlenstoff erhöht im Gegensatz zu Stickstoff die Bruch- und Gleichmaßdehnung. Des Weiteren vermeidet Kohlenstoff effektiver als Stickstoff die Entstehung von Delta-Ferrit. Zudem ist Kohlenstoff stärker als Stickstoff Austenit stabilisierend.

Vergleichend zu Stickstoff erzeugt Kohlenstoff einen geringeren Festigkeitsanstieg sowohl für die Zugfestigkeit $R_m$ als auch für Dehngrenze $R_{p0,2}$. Stickstoff führt durch eine größere Gitterverzerrung, einen wesentlich größeren Nahordnungseffekt und durch eine deutlich stärkere Feinkornhärtung zu höheren Festigkeiten. Der geringere Effekt des Kohlenstoffs auf die Feinkornhärtung ist in der erfindungsgemäßen Anwendung von besonderer Bedeutung, da sowohl ein feines Korn als auch eine niedrige Dehngrenze gefordert ist und diese gegensätzlichen Anforderungen bei einer ausreichend hohen Repassivierbarkeit erst durch die Zugabe von Kohlenstoff vereinbar werden.

Kohlenstoff erhöht vergleichbar mit Stickstoff die allgemeine Korrosionsbeständigkeit. Die Repassivierbarkeit jedoch wird durch die Zugabe von Kohlenstoff im besonderen Maße erhöht. Die Repassivierbarkeit von Stents, insbesondere von vaskulären Implantaten, ist von besonderer Wichtigkeit, da die Passivschicht beim Einsetzen des Implantats zerstört wird und die Oberfläche im sauerstoffarmen (sauerstoffarm in Bezug auf chemisch nicht gebundenen Sauerstoff) sowie korrosiven Medium Blut repassivieren muss.

Zusammengefasst lässt sich sagen, dass ein Ersetzen eines Teils des Stickstoffgehalts durch Kohlenstoff für die Anwendung als Stent und insbesondere als vaskuläre Gefäßstütze sowohl aus mechanischer Sicht, durch ein Erhöhen der Gleichmaß- und Bruchdehnung so wie durch eine Verringerung der Festigkeiten, als auch aus chemischer Sicht, durch ein Erhöhen des Repassivierungspotentials, von Vorteil ist.

Des Weiteren wird die Entstehung von Delta-Ferrit verhindert. Die hohe Bindungsbereitschaft des Kohlenstoffs stellt jedoch erhöhte metallurgische Anforderungen, da Titan, Niob und Vanadium bevorzugt nur in sehr geringen Gehalten von je kleiner als 0,02 % vorkommen sollten und der Kohlenstoffgehalt genau eingestellt werden muss.

Des Weiteren ist es von Vorteil die Parameter der Wärmebehandlung, die aus den Aufheizraten, den Abkühlraten, den Haltezeiten und den vorherrschenden Atmosphären bestehen in einer Weise zu erfüllen, dass in Abhängigkeit der vorliegenden Kaltverfestigungen und Bauteilabmessungen, die Entstehung von Karbiden ausgeschlossen werden können.

Bevorzugt liegt die Masse an Kohlenstoff im Bereich von 0,10 - 0,20 Gew.-%, vorzugsweise bei 0,12 - 0,20 Gew.-%, weiter bevorzugt 0,13 - 0,19 Gew.-%, noch weiter bevorzugt 0,14 - 0,18 Gew.-% und insbesondere bevorzugt von 0,15 - 0,17 Gew.-%.

Ferner ist bevorzugt, wenn die Summe der Gewichtsanteile an Stickstoff und Kohlenstoff in der Legierung von 0,7 - 0,90 Gew.-%, weiter bevorzugt 0,72 - 0,88 Gew.-% und noch weiter bevorzugt 0,73 - 0,86 Gew.-% und besonders bevorzugt 0,74 - 0,84 Gew.-% beträgt.

Zudem ist bevorzugt, wenn das Verhältnis der Gew.-% von Stickstoff und Kohlenstoffen in den folgenden Bereichen liegt: N : C von 3,0 bis 6,6, bevorzugt N : C von 3,3 bis 6,3 und weiter bevorzugt N : C von 3,5 bis 6,0.

Beim Verhältnis von N : C in dem Bereich von 3,5 bis 6,0 wurden die am stärksten ausgeprägten positiven Effekte ermittelt. Wodurch diese Effekte ausgelöst werden ist im Detail noch nicht bekannt. Es wird davon ausgegangen, dass ein höherer Kohlenstoffanteil durch Ausscheidungseffekte den positiven Effekt des atomar gelösten Kohlenstoffs, vor allem auf das Repassivierungsverhalten, überkompensiert.

Zusätzlich zu den zuvor genannten Bestandteilen kann eine erfindungsgemäße Stahllegierung auch neben Silizium noch 0,0 Gew.-% - 1,1 Gew.-%, bevorzugt 0,1 Gew.-% - 0,6 Gew.-%, weiter bevorzugt 0,2 Gew.-% - 0,4 Gew.-% Verunreinigungen, wie z.B. andere Metalle, Metallsalze, Nichtmetalle, Schwefel, Phosphor, Sauerstoff und/oder Wasserstoff enthalten. Diese weiteren Bestandteile sind zumeist herstellungsbedingte Verunreinigungen, welche in den vorgenannten geringen Mengen unschädlich für die Produkteigenschaften oder die Eigenschaften der Legierung sind. Bevorzugt ist jedoch, dass das Metall Kupfer (Cu) unter 300 ppm, bevorzugt unter 200 ppm und weiter bevorzugt unter 150 ppm vorhanden ist.

Des Weiteren ist bevorzugt, dass die Anteile der Metalle Vanadium und Kobalt jeweils ≤ 0,02 Gew.-% bevorzugt ≤ 0,01 Gew.-% weiter bevorzugt ≤ 0,005 Gew.-% sind.

Eine bevorzugte Zusammensetzung einer erfindungsgemäßen Stahllegierung umfasst oder besteht aus:

16,0 Gew.-% Chrom
12,0 Gew.-% Mangan
3,19 Gew.-% Molybdän
0,62 Gew.-% Stickstoff
0,15 Gew.-% Kohlenstoff
< 0,03 Gew.-% Nickel
bis 0,10 Gew.-% Verunreinigungen, wie z.B. andere Metalle und/oder
andere Nichtmetalle
bis auf 100 Gew.-% Eisen.

Eine weitere bevorzugte Zusammensetzung einer erfindungsgemäßen Stahllegierung umfasst oder besteht aus:

16,5 Gew.-% Chrom
10,0 Gew.-% Mangan
3,60 Gew.-% Molybdän
0,68 Gew.-% Stickstoff
0,17 Gew.-% Kohlenstoff
<0,03 Gew.-% Nickel
bis 0,10 Gew.-% Verunreinigungen, wie z.B. andere Metalle und/oder
andere Nichtmetalle
bis auf 100 Gew.-% Eisen.

Eine weitere bevorzugte Zusammensetzung einer erfindungsgemäßen Stahllegierung umfasst oder besteht aus:

16,05 Gew.-% Chrom
12,0 Gew.-% Mangan
3,21 Gew.-% Molybdän
0,63 Gew.-% Stickstoff
0,14 Gew.-% Kohlenstoff
0,06 Gew.-% Nickel
0,82 Gew.-% Silizium
bis 0,10 Gew.-% Verunreinigungen, wie z.B. andere Metalle und/oder
andere Nichtmetalle
bis auf 100 Gew.-% Eisen.

Alle in dieser Offenbarung angegebenen Gew.% beziehen sich auf das Gesamtgewicht der entsprechenden Legierung. Somit gilt bei allen hierin aufgeführten Zusammensetzungen, dass die Summe aller Bestandteile insgesamt 100,00 Gew.-% ergeben muss. Das heißt, dass nach Addition aller aufgeführten Komponenten der Eisenlegierung die Differenz zu 100 Gew.-% aus Eisen als Hauptbestandteil besteht. Darüber hinaus können diese Zusammensetzungen noch einen sehr geringen Anteil an teilweise nicht vermeidbaren, herstellungsbedingten Verunreinigungen enthalten. Wenn die Menge der Verunreinigungen nicht explizit angeführt ist, ist es bevorzugt, dass diese Verunreinigungen jeweils ≤ 0,2 Gew.-%, insbesondere ≤ 0,02 Gew.-% und in der Summe aller Verunreinigungen ≤ 1,0 Gew.-%, weiter bevorzugt ≤ 0,6 Gew.-% sind.

Ferner umfasst die vorliegende Erfindung bevorzugt Stahllegierungen, die neben Eisen, Chrom, Mangan, Molybdän, Stickstoff, Kohlenstoff und nicht vermeidbaren, herstellungsbedingten Verunreinigungen wie z.B. Nickel, Phosphor, Silizium, Schwefel keine weiteren Bestandteile umfassen. Das heißt, es ist bevorzugt, wenn die Bestandteile der Legierung, neben der Basis Eisen, ausgewählt werden aus der folgenden Gruppe bestehend oder umfassend aus: Chrom, Mangan, Molybdän, Stickstoff, Kohlenstoff und nicht vermeidbaren, herstellungsbedingten Verunreinigungen. Es ist insbesondere bevorzugt, dass die erfindungsgemäßen Legierungen kein Kupfer enthalten. Da Kupfer nicht nur vermehrt zur Apoptose, sondern auch zu nekrotischem Zelltod mit Entzündungserscheinungen und Abszessen führt soll der Kupfergehalt so weit wie technisch möglich limitiert werden und nicht mehr als 0,02 Gew.-% und weiter bevorzugt nicht mehr als 500 ppm, weiter bevorzugt nicht mehr als 300 ppm und insbesondere bevorzugt nicht mehr als 150 ppm betragen. Es ist beschrieben, dass schon geringste Kupfergehalte von 0,1 x 10-3 Mol proliferationshemmend wirken. Dieser Effekt kann für manche Anwendungen erwünscht sein bei denen das Einwachsen des Implantats oder zumindest von Teilen des Implantats unerwünscht ist. Beispielhaft sind hier Gelenkflächen von künstlichen Gelenken zu nennen. In der Anwendung als vaskulärer Stent hat eine dauerhafte Proliferationshemmung jedoch den wesentlichen Nachteil, dass der Stent in direktem Blutkontakt bleibt und auch längere Zeit nach dem Einbringen des Stents eine Thrombose auftreten kann. Eine Thrombose gilt es aufgrund der resultierenden hohen Mortalitätsrate so weit wie möglich zu vermeiden. Daher müssen auch Spuren von Kupfer so weit wie technisch möglich verringert werden.

Vergleichbar mit Nickel ist auch Kobalt als häufiges Kontaktallergen bekannt, so dass die Menge an Kobalt in der Legierung auf das technisch mögliche Minimum reduziert sein soll. Es ist daher insbesondere bevorzugt, dass die erfindungsgemäßen Legierungen kein Kobalt enthalten. Zumindest jedoch soll die maximale Menge von Kobalt in einer der erfindungsgemäßen Legierungen ≤ 0,2 Gew.-%, bevorzugt ≤ 0,05 Gew.-%, bevorzugt ≤ 0,02 Gew.-%, und weiter bevorzugt ≤ 500 ppm, weiter bevorzugt ≤ 300 ppm und insbesondere bevorzugt ≤ 150 ppm sein.

Vanadium bildet in Stahllegierungen besonders stabile Carbide aus, welche erfindungsgemäß möglichst vermieden werden sollen. Vanadiumcarbid ist zudem als krebserzeugend und keimzellmutagen jeweils in die Kategorie 2 eingestuft. Es ist daher insbesondere bevorzugt, dass die erfindungsgemäßen Legierungen kein Vanadium enthalten. Zumindest jedoch soll die maximale Menge von Vanadium in einer der erfindungsgemäßen Legierungen ≤ 0,2 Gew.-%, bevorzugt ≤ 0,05 Gew.-% und weiter bevorzugt ≤ 0,02 Gew.-%, bevorzugt ≤ 500 ppm, weiter bevorzugt ≤ 300 ppm und insbesondere bevorzugt ≤ 150 ppm sein.

Mit Gold beschichtete Stents haben in klinischen Studien schlechtere Resultate ergeben als unbeschichtete Stahlstents, so dass die erfindungsgemäße Legierung kein Gold enthalten soll. Bevorzugt ist der Goldgehalt in einer der erfindungsgemäßen Legierungen daher ≤ 0,2 Gew.-%, weiter bevorzugt ≤ 0,05 Gew.-% und noch weiter bevorzugt ≤ 0,02 Gew.-% und weiter bevorzugt ≤ 500 ppm, weiter bevorzugt ≤ 300 ppm und insbesondere bevorzugt ≤ 150 ppm

Falls herstellungsbedingte Verunreinigungen neben Eisen, Chrom, Mangan, Molybdän, Stickstoff, Kohlenstoff, in der Legierung enthalten sind, so handelt es sich bei diesen herstellungsbedingte Verunreinigungen um andere Metalle, Metallsalze; Nichtmetalle, Silizium, Schwefel, Nickel, Titan, Niob, Phosphor und/oder Wasserstoff, welche in geringen Mengen von < 2,00 Gew.-%, bevorzugt < 1,10 Gew.-%, bevorzugt < 0,80 Gew.-%, weiter bevorzugt < 0,60 Gew.-%, weiter bevorzugt < 0,50 Gew.-%, weiter bevorzugt < 0,40 Gew.-%, weiter bevorzugt < 0,30 Gew.-%, weiter bevorzugt < 0,20 Gew.-% und insbesondere bevorzugt < 0,10 Gew.-% vorhanden sind.

Als "andere Metalle", welche in der Zusammensetzung der erfindungsgemäßen Eisenlegierung als Verunreinigungen vorhanden sein können, sind die folgenden zu nennen: Beryllium, Natrium, Aluminium, Kalium, Calcium, Scandium, Titan, Magnesium, Gallium, Niobium, Technetium, Ruthenium, Rhodium, Palladium, Silber, Indium, Dysprosium, Neodym, Gallium, Gadolinium, Yttrium, Lithium, Zink, Zirkonium, Zinn, Lanthan, Cer, Praseodym, Promethium, Samarium, Terbium, Holmium, Erbium, Thulium, Lutetium, Tantal, Rhenium, Platin und Blei. Ferner können Metallsalze in sehr geringen Mengen als Verunreinigung in der Legierung enthalten sein.

Metallsalze enthalten vorzugsweise mindestens eines der folgenden Metallionen: $Be^{2+}$, $Na^+$, $Mg^{2+}$, $K^+$, $Ca^{2+}$, $Sc^{3+}$, $Ti^{2+}$, $Ti^{4+}$, $Cr^{2+}$, $Cr^{3+}$, $Cr^{4+}$, $Cr^{6+}$, $Mn^{2+}$, $Mn^{3+}$, $Mn^{4+}$, $Mn^{5+}$, $Mn^{6+}$, $Mn^{7+}$, $Fe^{2+}$, $Fe^{3+}$, $Ni^{2+}$, $Zn^{2+}$, $Al^{3+}$, $Zr^{2+}$, $Zr^{4+}$, $Nb^{2+}$, $Nb^{4+}$, $Nb^{5+}$, $Mo^{4+}$, $Mo^{6+}$, $Tc^{2+}$, $Tc^{3+}$, $Tc^{4+}$, $Tc^{5+}$, $Tc^{s+}$, $Tc^{7+}$, $Ru^{3+}$, $Ru^{4+}$, $Ru^{5+}$, $Ru^{6+}$, $Ru^{7+}$, $Ru^{8+}$, $Rh^{3+}$, $Rh^{4+}$, $Pd^{2+}$, $Pd^{3+}$, $Ag^+$, $In^+$, $In^{3+}$, $Ta^{4+}$, $Ta^{5+}$, $Pt^{2+}$, $Pt^{3+}$, $Pt^{4+}$, $Pt^{5+}$, $Pt^{6+}$, $Au^+$, $Au^{3+}$, $Au^{5+}$, $Sn^{2+}$, $Sn^{4+}$, $Pb^{2+}$, $Pb^{4+}$, $La^{3+}$, $Ce^{3+}$, $Ce^{4+}$, $Gd^{3+}$, $Nd^{3+}$, $Pr^{3+}$, $Tb^{3+}$, $Pr^{3+}$, $Pm^{3+}$, $Sm^{3+}$, $Eu^{2+}$, $Dy^{3+}$, $Ho^{3+}$, $Er^{3+}$, $Tm^{3+}$.

Als Anionen dienen Halogene wie $F^-$, $Cl^-$, $Br^-$, Oxide und Hydroxide wie $OH^-$, $O^{2-}$, Sulfate, Carbonate, Oxalate, Phosphate wie $HSO4^-$, $SO_4^{2-}$, $HCO_3^-$, $CO_3^{2-}$, $HC_2O_4^-$, $C_2O_4^{2-}$, $H_2PO_4^-$, $HPO_4^{2-}$, $PO_4^{3-}$, und insbesondere Carboxylate wie $HCOO^-$, $CH_3COO^-$, $C_2H_5COO^-$, $C_3H_7COO^-$, $C_4H_9COO^-$, $C_5H_{11}COO^-$, $C_6H_{13}COO^-$, $C_7H_{15}COO^-$, $C_8H_{17}COO^-$, $C_9H_{19}COO^-$, $PhCOO^-$, $PhCH_2COO^-$.

Des Weiteren sind Salze der folgenden Säuren möglich: Schwefelsäure, Sulfonsäure, Phosphorsäure, Salpetersäure, salpetrige Säure, Perchlorsäure, Bromwasserstoffsäure, Chlorwasserstoffsäure, Ameisensäure, Essigsäure, Propionsäure, Bernsteinsäure, Oxalsäure, Gluconsäure (Glycons., Dextronsäure), Milchsäure, Äpfelsäure, Weinsäure, Tartronsäure (Hydroxymalonsäure, Hydroxypropandisäure), Fumarsäure, Zitronensäure, Ascorbinsäure, Maleinsäure, Malonsäure, Hydroxymaleinsäure, Brenztraubensäure, Phenylessigsäure, (o-, m-, p-) Toluylsäure, Benzoesäure, p-Aminobenzoesäure, p-Hydroxybenzoesäure, Salicylsäure, p-Aminosalicylsäure, Methansulfonsäure, Ethansulfonsäure, Hydroxyethansulfonsäure, Ethylensulfonsäure, p-Toluolsulfonsäure, Naphthylsulfonsäure, Naphthylaminsulfonsäure, Sulfanilsäure, Camphersulfonsäure, Chinasäure, Chininsäure, o-Methyl-mandelsäure, Hydrogenbenzolsulfonsäure, Methionin, Tryptophan, Lysin, Arginin, Pikrinsäure (2,4,6-Trinitrophenol), Adipinsäure, d-o-Tolylweinsäure, Glutarsäure.

Um die mechanischen Eigenschaften der Stahllegierung zu optimieren ist es bevorzugt, dass die Zusammensetzung einer Wärmebehandlung unterzogen wird.

Einstellbare Parameter bei der Wärmebehandlung sind der Temperaturverlauf sowie der vorherrschende Druck und die Gaszusammensetzung. Der Temperaturverlauf lässt sich in die Aufheiz- und Abkühlraten und in die Haltezeiten unterteilen.

Die bei der Wärmebehandlung einstellbaren Parameter und die Parameter der Legierung Wechselwirken in unterschiedlichen Weisen in Bezug auf das erzeugte Ergebnis.

Beispielsweise erhöht ein hoher Stickstoffgehalt die Festigkeit der Legierung, so dass dieses durch die Wärmebehandlung kompensiert werden muss. Der Stickstoffgehalt der Legierung hängt auch von den Gehalten der Legierungselemente ab, die die Stickstofflöslichkeit erhöhen oder verringern, dieses muss zum Einstellen des Stickstoffgehalts durch eine Druckanpassung kompensiert werden. Des Weiteren ist die Quadratwurzel des Gasdrucks proportional zum resultierenden Stickstoffgehalt (Sievert's Law) und die Stickstofflöslichkeit ist eine Funktion der Temperatur. Somit ist der Stickstoffgehalt von der Legierungszusammensetzung, dem Prozessdruck und von der Temperatur abhängig.

Des Weiteren hat der Grad der Versetzungsdichte einen Einfluss auf die Kornentstehungsgeschwindigkeit und vorliegende Mikroausscheidungen beeinflussen das Kornwachstum.

Anhand der Beispielhaft genannten Parameter, die sich gegenseitig beeinflussen, wird deutlich, dass die Wärmebehandlungsparameter an die jeweiligen Voraussetzungen angepasst werden müssen.

Aufgrund der geringen Wandstärke der Stents ist es möglich im ganzen Bauteil einen starken Temperaturgradienten über die Zeit zu erzeugen.

In den Aufheizphasen werden Temperaturanstiege zwischen 200°C und 500°C pro Minute erzeugt und in den Abkühlphasen werden Temperaturdifferenzen von größer 3000°C und bevorzugt größer 5000°C pro Minute erzeugt.

Die maximale Bauteiltemperatur liegt im Bereich von 1050°C bis 1250°C und die Haltezeit beträgt zwischen 15 und 45 Minuten.

Die Wärmebehandlungen werden unter Stickstoffatmosphäre durchgeführt. Die Drücke wurden so gewählt, dass der gewünschte Stickstoffgehalt in der Legierung erreicht wird. Die verwendeten Drücke liegen im Bereich von 500 mbar bis 2500 mbar.

Die Korngröße nach EN ISO 643 ist hierin definiert als durchschnittliche Größe einzelner Kristalle innerhalb eines Metalls bzw. einer Legierung, bei welchen die Kristalle auch als Körner bezeichnet werden. Die Korngröße hat dabei auch Auswirkungen auf die physikalischen Eigenschaften der Legierung: Ein feinkörniges Gefüge verleiht der Legierung hohe Festigkeit und Dehnbarkeit.

Eine kurze Wärmebehandlung wie oben beschrieben erzeugt ein feinkörniges Gefüge. Ein feinkörniges Gefüge ist auf Grund der bevorzugten Materialstärken von etwa 100 $\mu$m von besonderer Relevanz. Eine bevorzugte Korngröße liegt bei G = 6 - 10 und insbesondere bevorzugt bei ungefähr G = 7 - 8, was etwa 7 - 10 Körnern auf 100 $\mu$m entspricht.

Die Zugfestigkeit $R_m$ bezeichnet die Grenze, an der der Stahl bei Belastung reißt, also die maximale Zugspannung des Stahls. Die Zugfestigkeit wird durch den Zugversuch ermittelt. Die Zugfestigkeit wird mit dem Kurzzeichen $R_m$ bezeichnet.

Die Bruchdehnung A ist ein Materialkennwert, der die bleibende Verlängerung der Probe nach dem Bruch, bezogen auf die Anfangslänge, angibt. Die Bruchdehnung charakterisiert die Verformungsfähigkeit (bzw. Duktilität) eines Werkstoffes. Angegeben wird hierin die erfolgte Verlängerung einer Probe der Legierung nach dem Bruch (in %), bezogen auf die ursprüngliche Probenlänge.

Es ist bevorzugt, wenn die Bruchdehnung der erfindungsgemäßen Stahllegierung größer 60 % ist und weiter bevorzugt, wenn die Bruchdehnung größer 65 % ist.

Die Dehngrenze $R_p$ ist hierin definiert als die Spannung, bei der nach Entlastung eine bleibende Dehnung festgestellt (= 0,2 % bleibende Verformung) wird. Die gemessene Verformung wird als Index angegeben, der hierin verwendete Wert ist 0,2 % ($R_{p0,2}$).

Es ist bevorzugt, wenn die mit dem oben beschriebenen Wärmebehandlungsverfahren erzeugte Dehngrenze $R_{p0,2}$ der Stahllegierungen zwischen 500 und 600 MPa liegt.

Des Weiteren ist bevorzugt, dass die Zugfestigkeit $R_m$ zwischen 900 und 1200 MPa liegt.

Die erfindungsgemäßen Stahllegierungen sind insbesondere als Werkstoff zur Herstellung von Endoprothesen bzw. Stents geeignet.

Des Weiteren umfasst die vorliegende Erfindung daher einen Stent bestehend aus einer der hier offenbarten Stahllegierungen. Es handelt sich bei dem erfindungsgemäßen Stent bevorzugt um einen Stent für Blutgefäße, Harnwege, Atemwege, Gallenwege oder den Verdauungstrakt. Unter diesen Stents sind wiederum die Stents für Blutgefäße oder allgemeiner für das Herz-Kreislaufsystem besonders bevorzugt.

Die Stents werden vorzugsweise per Laser aus einem Rohr geschnitten, welches aus einer erfindungsgemäßen Eisenlegierung besteht. Unter Stents werden hierbei gitterförmige oder netzförmige Endoprothesen verstanden, die in ein Hohlorgan oder einen Körperhohlraum eingesetzt werden, um diesen offen zu halten. Ein Stent bildet kein massives Rohr, sondern ein Gittergeflecht. Betrachtet man beispielsweise einen vaskulären Stent, so wird dieser aus einem massiven Rohr z.B. mittels Laser geschnitten, so dass sich einzelne, möglichst dünne Streben ergeben, welche untereinander verbunden sind. Die Anordnung der Streben und Knotenpunkte wird als Stentdesign bezeichnet und kann erfindungsgemäß variieren.

Beim Schneiden eines Stents werden Flächen zwischen den einzelnen Streben herausgeschnitten. Eine Endoprothese weist daher eine Vielzahl von massiven Gerüstkomponenten (z.B. Streben in Form von Ringen, Spiralen, Wellen und Drähten) auf, welche insgesamt die Endoprothese bilden sowie eine Vielzahl von Zwischenräumen zwischen diesen massiven Komponenten. Bei der gängigen Ausführungsform von Endoprothesen laufen die Streben in Knotenpunkten zusammen. Es gibt jedoch auch Ausführungsformen von Endoprothesen, bei denen keine oder fast keine Knotenpunkte vorhanden sind und die Streben z.B. die Form von Ringen oder Spiralen haben. Bevorzugt handelt es sich um selbstexpandierbare oder ballonexpandierbare Stents, welche mittels Katheter bis zur erkrankten oder zu behandelnden Stelle geschoben werden, wo die Stents auf ihren definierten Normdurchmesser aufgeweitet werden.

Die Stents werden aus Röhren mittels Laser geschnitten, die aus einer erfindungsgemäßen Legierung bestehen. Die Rohre werden durch Umformung von Drähten aus den erfindungsgemäßen Legierungen erhalten.

## Beispiele

### Beispiel 1: Herstellung der Legierungen

Als Einsatzmaterialien werden zur Erzeugung der Vorlegierung reinste Ausgangswerkstoffe verwendet und in einer Vakuumschmelzanlage aufgeschmolzen. Dabei werden alle Legierungsbestandteile, abgesehen vom Stickstoff, der Legierung in den entsprechenden Mengen zugefügt.

Das Ausgangsmaterial wird mittels DESU-Verfahren (Druck-Elektro-Schlacke-Umschmelzen) umgeschmolzen, wobei der Stickstoffgehalt eingestellt wird.

### Beispiel 2: Rohrherstellung

Aus den Legierungen die wie in Beispiel 1 beschrieben hergestellt wurden, wurde ein an die Strangpresse angepasster Rohling vor dem Strangpressen für 3-6 Stunden in reduzierender Stickstoffatmosphäre auf 1100 °C bis 1250 °C erwärmt und nach dem Strangpressen an Luft abgekühlt. Die erzeugten Stränge wurden mittels Präzisionsböhrverfahren zentrisch hohl gebohrt. Es folgten Ziehschritte mit jeweils einer anschließenden Wärmebehandlung, in reduzierender Stickstoffatmosphäre bei 1100 °C bis 1250 °C, in denen das Rohr auf Nennmaß umgeformt wurde.

### Beispiel 3: Stentfertigung

Ein gemäß Beispiel 2 hergestelltes Rohr wird in eine Aufnahme in der Lasermaschine fixiert. Ein gepulster Festkörperlaser (FKL) schneidet aus dem Rohr die Konturen des Stentdesigns heraus. Das Laserschneiden erfolgt unter Schutzgasatmosphäre.

Das Stentdesign ist in einem NC-Programm (numerical control = Numerische Steuerung) hinterlegt. Dieses gibt dem Laser die Verfahrwege vor, nach denen das Rohr strukturiert wird. Durch das Laserstrahlschneiden kommt es zur Gradbildung, insbesondere auf der Rohrinnenseite, entlang der gesamten Schneidkontur. Dies kann dazu führen, daß Reststücke und Ausschnitte nach Beendigung des Schneidvorgangs in der Kontur haften bleiben. Die Reststücke und Ausschnitte werden mechanisch entfernt und der Stent von Fertigungsrückständen gereinigt. In einer ersten optischen Sichtkontrolle wird eine Inspektion der Schneidkontur durchgeführt.

Im Folgenden wird der Stent elektro-chemisch poliert. Der Stent wird anodisch beschaltet und in ein Säurebad getaucht. Über eine im Bad fixierte Kathode wird ein Stromkreis geschlossen. Der Stromkreis bleibt für mehrere Minuten aufrechterhalten. Die Elektropolitur ist ein umgekehrter galvanischer Prozess bei dem kontrolliert Material von der Oberfläche des anodisch beschalteten Bauteils abgetragen wird. Dabei wird durch das Verfahren bedingt bevorzugt an scharfkantigen Ecken und Kanten abgetragen. Der Stent erhält eine glatte Oberfläche und abgerundete Kanten entlang der Konturen. Nach der Politur wird der Stent gereinigt und von Säurerückständen befreit. In der Endreinigung werden alle noch verbliebenen Fertigungsrückstände von der Stentoberfläche entfernt. In einer letzen optischen Sichtkontrolle wird die Stentgeometrie vermessen und die Oberfläche auf Reinheit geprüft.

### Beispiel 4: Ermittlung des optimalen Chromgehalts in einer erfindungsgemäßen Legierung

Zur Ermittlung eines optimal eingestellten Chromgehalts wurden gemäß Beispiel 1 die Legierungen A - I hergestellt, welche die folgenden Zusammensetzungen aufweisen:

|     | A    | B    | C    | D    | E    | F    | G    | H    | I    |
|-----|------|------|------|------|------|------|------|------|------|
| Cr  | 12,0 | 13,0 | 14,0 | 15,5 | 16,0 | 16,5 | 17,0 | 17,5 | 18,0 |
| Mn  | 11   | 11   | 11   | 11   | 11   | 11   | 11   | 11   | 11   |
| Mo  | 3,19 | 3,19 | 3,19 | 3,19 | 3,19 | 3,19 | 3,19 | 3,19 | 3,19 |
| N   | 0,62 | 0,62 | 0,62 | 0,62 | 0,62 | 0,62 | 0,62 | 0,62 | 0,62 |
| C   | 0,15 | 0,15 | 0,15 | 0,15 | 0,15 | 0,15 | 0,15 | 0,15 | 0,15 |
| Ni  | 0,03 | 0,03 | 0,03 | 0,03 | 0,03 | 0,03 | 0,03 | 0,03 | 0,03 |
| Si  | 0,33 | 0,33 | 0,33 | 0,33 | 0,33 | 0,33 | 0,33 | 0,33 | 0,33 |
| P   | 0,01 | 0,01 | 0,01 | 0,01 | 0,01 | 0,01 | 0,01 | 0,01 | 0,01 |

Das Korrosionsverhalten wurde anhand von Stents mittels potentiostatischer Untersuchungen ermittelt.

Die potentiostatischen Untersuchungen-wurden in einer sauerstofffreien gepufferten physiologischen Lösung bei 37 °C durchgeführt. Es wird zuerst das Ruhepotential ermittelt, das heißt, dass eine Referenzelektrode und das Bauteil ohne angelegte Spannung in die Lösung getaucht werden. Es stellt sich eine Potentialdifferenz ein, die sich über die Zeit ändert. Anhand der Potentialdifferenz, die sich nach einer Stunde eingestellt hat kann eine erste Aussage über die Beständigkeit der Legierung gemacht werden. Die Legierung ist umso beständiger je positiver der Wert ist.

Anschließend wurde eine zyklische potentiodynamische Polarisation durchgeführt. Dazu wurde eine Potentialdifferenz zwischen das Bauteil und die Referenzelektrode angelegt. Das Anfangspotential wird so gewählt, dass es 0,1 mV geringer ist als das sich eingestellte Ruhepotential. Das angelegte Potential wird über die Zeit auf z.B. 1,2 Volt erhöht und dann auf den Ausgangswert verringert, wobei der sich ergebende Strom gemessen wird. Anhand der Polarisationskurve, die eine Spannungsstromkurve ist, können sowohl die Korrosionsraten, die minimalen Ströme, die Durchbruchspotentiale als auch die Repassivierungspotentiale bestimmt werden. Die Parameter wurden nach ASTM F2129-10 mit PBS (Phosphat buffered saline) ermittelt.

Für die erfindungsgemäßen Legierungen wurden Korrosionsraten zwischen 15 und 25 nm/y (Nanometer pro Jahr) gemessen. Für weniger beständige Legierungen wurden Korrosionsraten von größer als 50 nm/y ermittelt.

Die ermittelten Durchbruchpotentiale für die erfindungsgemäßen Legierungen liegen zwischen 1030 mV und 1070 mV. Im Gegensatz dazu ist das Durchbruchpotential bei weniger beständigen Legierungszusammensetzungen bereits bei 800 mV erreicht.

Noch wesentlicher ist der Unterschied beim Repassivierungsverhalten, welchem bei der Anwendung als Stent eine besondere Bedeutung zukommt. Die erfindungsgemäßen Legierungen weisen ein Repassivierungspotential von 940 bis 960 mV auf wobei Legierungen mit einem geringen Repassivierungspotential lediglich ein Repassivierungspotential von 100 bis 150 mV erreichen.

Die ermittelten Werte haben jeder für sich betrachtet nur eine relativ geringe Aussagekraft, erst die Kombination von guten Einzelwerten ergeben ein gutes Korrosionsverhalten, wobei die Gewichtung der Einzelwerte vom Anwendungsfall abhängt.

Die Legierungen A und B weisen im polierten Zustand eine unebene Oberfläche auf. Lichtmikroskopisch betrachtet weist die Oberfläche leicht matte Stellen auf und erscheint insgesamt nicht hoch glänzend. Die Korrosionsbeständigkeit und insbesondere das Repassivierungspotential sind gegenüber den Legierungen C bis E verringert.

Die Legierungen C bis E weisen ein sehr gutes Korrosionsverhalten auf. Die chemische Beständigkeit ist deutlich höher als bei dem für vaskuläre Stents verwendeten Material 1.4441. Die Durchbruch- und Repassivierungspotentiale sind vergleichbar mit dem Material 2.4964 (L605).

Die Legierungen C bis E weisen eine hervorragende Polierbarkeit auf. Es wird eine fehlerfreie Oberfläche ohne messbare Welligkeiten und ohne Vertiefungen oder Erhöhungen erzeugt. Lichtmikroskopisch betrachtet liegt eine hochglänzende Oberfläche vor. Die Legierung F zeigt eine gute Polierbarkeit, die Oberfläche weist jedoch vereinzelt Vertiefungen auf, die teilweise nicht auspoliert sind. Das Korrosionsverhalten ist noch ausreichend, vergleichbar mit dem Material 1.4441.

Die Legierungen G bis I weisen eine mit steigendem Chromgehalt zunehmend schlechtere Polierbarkeit auf. Die Politur erzeugt eine wellige Oberfläche mit nicht auspolierten Vertiefungen.

Potentiostatische Untersuchungen an den Legierungen G bis I weisen ein verringertes Durchbruchpotential und ein deutlich verringertes Repassivierungspotential auf. Die nicht erwartete Verschlechterung der Polierbarkeit und Verringerung der Korrosionsbeständigkeit bei Erhöhung des Chromgehalts wird auf die Entstehung von Sigmaphasen und Deltaferrit zurückgeführt. Sigmaphasen und Deltaferrit entstehen bei Temperaturen von etwa 600°C - 800°C und können durch die Wärmebehandlung erzeugt worden sein. Da die Wärmebehandlung sowohl eine niedrige Dehngrenze als auch eine hohe Dehnbarkeit und ein kleines Korn von G > 7 sicherstellen muss, lässt sich die Entstehung von Sigmaphasen und/oder Deltaferrit für Chromgehalte größer als 16,5 % nicht vermeiden. Der Chromgehalt sollte daher auf 16,5 % begrenzt werden. Aufgrund der Verbesserung der Korrosions- und Poliereigenschaften sollte zudem Chrom einen minimalen Bestandteil von 14 % an der Legierung ausmachen.

**Beispiel 5: Ermittlung des optimalen Mangangehalts in einer erfindungsgemäßen Legierung**

Zur Ermittlung eines optimal eingestellten Mangangehalts wurden gemäß Beispiel 1 die Legierungen A - O hergestellt, welche die folgenden Zusammensetzungen aufweisen:

|     | A    | B    | C    | D    | E    | F    | G    | H    | I    | J    | K    | L    |
|-----|------|------|------|------|------|------|------|------|------|------|------|------|
| Cr  | 16,0 | 16,0 | 16,0 | 16,0 | 16,0 | 16,0 | 16,0 | 16,0 | 16,0 | 16,0 | 16,0 | 16,0 |
| Mn  | 10   | 11   | 11,6 | 11,8 | 12   | 12,2 | 12,4 | 12,6 | 12,8 | 13   | 14   | 16   |
| Mo  | 3,19 | 3,19 | 3,19 | 3,19 | 3,19 | 3,19 | 3,19 | 3,19 | 3,19 | 3,19 | 3,19 | 3,19 |
| N   | 0,62 | 0,62 | 0,62 | 0,62 | 0,62 | 0,62 | 0,62 | 0,62 | 0,62 | 0,62 | 0,62 | 0,62 |
| C   | 0,15 | 0,15 | 0,15 | 0,15 | 0,15 | 0,15 | 0,15 | 0,15 | 0,15 | 0,15 | 0,15 | 0,15 |
| Ni  | 0,03 | 0,03 | 0,03 | 0,03 | 0,03 | 0,03 | 0,03 | 0,03 | 0,03 | 0,03 | 0,03 | 0,03 |
| Si  | 0,33 | 0,33 | 0,33 | 0,33 | 0,33 | 0,33 | 0,33 | 0,33 | 0,33 | 0,33 | 0,33 | 0,33 |
| P   | 0,01 | 0,01 | 0,01 | 0,01 | 0,01 | 0,01 | 0,01 | 0,01 | 0,01 | 0,01 | 0,01 | 0,01 |

|     | M    | N    | O    |
|-----|------|------|------|
| Cr  | 16,0 | 16,0 | 16,0 |
| Mn  | 18   | 20   | 22   |
| Mo  | 3,19 | 3,19 | 3,19 |
| N   | 0,62 | 0,62 | 0,62 |
| C   | 0,15 | 0,15 | 0,15 |
| Ni  | 0,03 | 0,03 | 0,03 |
| Si  | 0,33 | 0,33 | 0,33 |
| P   | 0,01 | 0,01 | 0,01 |

Die mechanischen Parameter Dehngrenze $R_{p0,2}$, Zugfestigkeit $R_m$ und Bruchdehnung (A) wurden im Zugversuch an Rohrproben nach DIN EN 10002-1 ermittelt. Dazu wurden Rohrproben zwischen zwei Halterungen gespannt. Die Halterungen wurden an der Zugprüfmaschine angebracht und die Zugprüfmaschine dehnt die Probe über die Länge bis zum Bruch. Die mechanischen Parameter werden anhand der gemessenen Kräfte und Strecken und der angegebenen Probengeometrie von der Maschine berechnet und ausgegeben.

Die Legierungen A, B, C und D weisen eine sehr gute Polierbarkeit auf. Es wird eine sehr hohe Oberflächengüte ohne messbare Welligkeiten und ohne Vertiefungen oder Erhöhungen erzeugt. Lichtmikroskopisch betrachtet liegt eine fehlerfreie hochglänzende Oberfläche vor. Insbesondere die Legierungen A und B zeigen im polierten Zustand eine hervorragende und absolut fehlerfreie lichtmikroskopisch betrachtet hochglänzende Oberfläche auf.

Die Dehngrenzen betragen für die Legierung A ca. 550 MPa und steigen für die Legierung D auf ca. 600 MPa. Die Bruchdehnungen dieser Legierungen betragen bis über 65 %.

Die Legierung E weist eine geringfügig schlechtere Polierbarkeit auf. Lichtmikroskopisch betrachtet liegt eine fehlerarme glänzende Oberfläche vor. Vereinzelt sind Lichtmikroskopisch leichte Welligkeiten der Oberfläche erkennbar. Zum Teil sind auch einzelne Vertiefungen im Bauteil vorhanden. Die Dehngrenze beträgt etwa 610 MPa und die Bruchdehnung dieser Legierung beträgt um 60 %.

Die Legierungen F, G, H und I weisen mit aufsteigendem Mangangehalt eine immer schlechter werdende Polierbarkeit auf. Lichtmikroskopisch betrachtet liegen fehlerbehaftete mattglänzende Oberflächen vor. Die Oberfläche ist wellig. Es liegen viele Vertiefungen vor. Vor allem die Legierungen H und I weisen viele nicht polierte Vertiefungen auf. Die Dehngrenze steigt bei Legierung I bis auf circa 640 MPa an. Mit steigendem Mangangehalt verringert sich die Bruchdehnung bis auf unter 60 %.

Die Legierungen J, K und L ermöglichen keine Erzeugung von polierten Oberflächen nach den Ansprüchen, die an Stents gestellt werden. Die Oberflächen wirken mit dem bloßen Auge betrachtet leicht matt, was auf nicht polierte Vertiefungen zurückzuführen ist. Die Dehngrenzen liegen bei Werten von bis über 760 MPa und die Bruchdehnung verringert sich auf bis etwa 40 %.

Die Legierungen M, N und O ermöglichen keine Erzeugung von polierten Oberflächen. Die Oberflächen wirken mit dem bloßen Auge betrachtet matt, was auf flächig vorliegende nicht polierte Vertiefungen zurückzuführen ist. Die Dehngrenzen liegen bei Werten von bis über 850 MPa und die Bruchdehnung verringert sich auf bis unter 35 %.

Des Weiteren wurden gemäß Beispiel 1 die Legierungen P - S hergestellt, welche die folgenden Zusammensetzungen aufweisen:

|     | P    | Q    | R    | S    |
|-----|------|------|------|------|
| Cr  | 16,0 | 16,0 | 16,0 | 16,0 |
| Mn  | 8,5  | 9,0  | 9,6  | 9,8  |
| Mo  | 3,19 | 3,19 | 3,19 | 3,19 |
| N   | 0,62 | 0,62 | 0,62 | 0,62 |
| C   | 0,15 | 0,15 | 0,15 | 0,15 |
| Ni  | 0,03 | 0,03 | 0,03 | 0,03 |
| Si  | 0,33 | 0,33 | 0,33 | 0,33 |
| P   | 0,01 | 0,01 | 0,01 | 0,01 |

Die Legierungen P und Q weisen eine Dehngrenze von ca. 500 MPa auf. Nach dem Polieren weisen die Oberflächen lichtmikroskopisch betrachtet kleinere Vertiefungen und vereinzelt auch Erhöhungen auf. Die Bruchdehnung erreicht Werte von etwa 50%.

Die Vertiefungen nach der Politur legen Ausscheidungseffekte im Material nahe. Dies deckt sich mit der zu Legierung A verringerten Bruchdehnung, da Ausscheidungen die Bruchdehnungen verringern. Da Mangan die Löslichkeit von atomar gelöstem Stickstoff erhöht, können Ausscheidungen entstehen, wenn bei konstantem Stickstoffgehalt der Mangangehalt verringert wird.

Die Legierung P weist nach dem Crimpen und Delatieren des Stents ein leicht ferritisches Verhalten auf. Die chemische Beständigkeit der Legierung P ist deutlich reduziert. Die Legierung Q weist eine verringerte chemische Beständigkeit auf.

Die Dehngrenzen der Legierungen R und S betragen etwa 540 MPa. Die Bruchdehnung erreicht Werte von ca. 60 %. Lichtmikroskopisch betrachtet ist die Oberflächengüte der Legierungen nach der Politur hoch und fehlerarm.

Die chemische Beständigkeit besonders von Legierung S ist nur im geringen Maße gegenüber der Legierung A verringert.

Die beste Oberflächengüte und die höchste Bruchdehnung sowie die geringste Dehngrenze wird bei den Legierungen A - E erzeugt, so dass der Mangangehalt der erfindungsgemäßen Stahllegierungen auf 10,0 % - 12,0 Gew.-% festgelegt wird.

**Beispiel 6: Untersuchung des Einflusses von Molybdän in einer erfindungsgemäßen Legierung**

Zur Untersuchung des Einflusses von Molybdän auf die mechanischen und chemischen Eigenschaften einer erfindungsgemäßen Legierung wurden gemäß Beispiel 1 die Legierungen A - N hergestellt, welche die folgenden Zusammensetzungen aufweisen:

|     | A    | B    | C    | D    | E    | F    | G    | H    | I    | J    | K    | L    | M    | N    |
|-----|------|------|------|------|------|------|------|------|------|------|------|------|------|------|
| Cr  | 16,0 | 16,0 | 16,0 | 16,0 | 16,0 | 16,0 | 16,0 | 16,0 | 16,0 | 16,0 | 16,0 | 16,0 | 16,0 | 16,0 |
| Mn  | 11   | 11   | 11   | 11   | 11   | 11   | 11   | 11   | 11   | 11   | 11   | 11   | 11   | 11   |
| Mo  | 5,0  | 4,5  | 4,2  | 4,0  | 3,8  | 3,6  | 3,4  | 3,2  | 3,0  | 2,8  | 2,6  | 2,4  | 2,2  | 2,0  |
| N   | 0,62 | 0,62 | 0,62 | 0,62 | 0,62 | 0,62 | 0,62 | 0,62 | 0,62 | 0,62 | 0,62 | 0,62 | 0,62 | 0,62 |
| C   | 0,15 | 0,15 | 0,15 | 0,15 | 0,15 | 0,15 | 0,15 | 0,15 | 0,15 | 0,15 | 0,15 | 0,15 | 0,15 | 0,15 |

(fortgesetzt)

|   | A | B | C | D | E | F | G | H | I | J | K | L | M | N |
|---|---|---|---|---|---|---|---|---|---|---|---|---|---|---|
| Ni | 0,03 | 0,03 | 0,03 | 0,03 | 0,03 | 0,03 | 0,03 | 0,03 | 0,03 | 0,03 | 0,03 | 0,03 | 0,03 | 0,03 |
| Si | 0,33 | 0,33 | 0,33 | 0,33 | 0,33 | 0,33 | 0,33 | 0,33 | 0,33 | 0,33 | 0,33 | 0,33 | 0,33 | 0,33 |
| P | 0,01 | 0,01 | 0,01 | 0,01 | 0,01 | 0,01 | 0,01 | 0,01 | 0,01 | 0,01 | 0,01 | 0,01 | 0,01 | 0,01 |

Die Legierungen A und B weisen eine schlechte Polierbarkeit auf. Lichtmikroskopisch betrachtet liegt eine mattglänzende Oberfläche vor. Die Oberfläche ist wellig. Es liegen sowohl Vertiefungen als auch Erhöhungen vor. Die Bruchdehnung beträgt zwischen 35 % - 40 %. Die Erhöhungen lassen sich durch die Entstehung von Karbiden begründen, da diese im Polierprozess verlangsamt abgetragen werden. Ebenso lassen sich die Vertiefungen auf Karbide zurückführen, da diese, wenn sie aus dem Material gelöst werden und aus dem Stent herausfallen, Vertiefungen zurück lassen. Die stark verringerte Bruchdehnung im Vergleich zu den Legierungen D bis I, lässt sich auf die Kerbwirkung von Karbiden und das an Kohlenstoff verarmte Material in der Umgebung der Karbide zurückführen.

Potentiostatische Untersuchungen zeigen einen höheren Flachabtrag und ein verschlechtertes Repassivierungsverhalten im Vergleich zu den Legierungen D bis I. Dies ist auf galvanische Korrosion zwischen Karbiden und Grundmaterial zurückzuführen.

Die Legierung C weist gegenüber den Legierungen A und B eine deutlich bessere Politur auf. Es liegt eine glänzende Oberfläche vor, die vereinzelt leichte Welligkeiten aufweist. Die Bruchdehnung beträgt über 50 %.

Die Legierungen D bis I weisen eine hervorragende Polierbarkeit auf. Es wird eine fehlerfreie Oberfläche ohne messbare Welligkeiten und ohne Vertiefungen oder Erhöhungen erzeugt. Lichtmikroskopisch betrachtet liegt eine hochglänzende Oberfläche vor. Die Bruchdehnungen dieser Legierungen betragen bis über 65 %.

Die Legierung J weist gegenüber den Legierung I eine geringfügig schlechtere Polierbarkeit auf. Lichtmikroskopisch betrachtet liegt eine fehlerarme glänzende Oberfläche vor. Vereinzelt sind Lichtmikroskopisch leichte Welligkeiten der Oberfläche erkennbar. Zum Teil sind auch einzelne Vertiefungen im Bauteil vorhanden. Die Bruchdehnung beträgt um die 50 %.

Potentiostatische Untersuchungen, wie unter Beispiel 4 beschrieben, an Legierung J ergeben ein im Vergleich zu den Legierungen D bis I geringfügig verringertes Repassivierungspotential. Die Legierungen K bis N weisen eine mit sinkendem Molybdängehalt zunehmend schlechtere Polierbarkeit auf. Die Politur erzeugt eine unebene Oberfläche mit nicht auspolierten Vertiefungen. Potentiostatische Untersuchungen an den Legierungen K bis N weisen ein verringertes Durchbruchpotential und ein deutlich verringertes Repassivierungspotential auf.

Der wesentliche Einfluss von Molybdän auf die Korrosionsbeständigkeit wird anhand des MARC-Wertes ersichtlich. Molybdän erhöht die chemische Beständigkeit 3,3-fach so stark wie Chrom.

$$MARC = [\%Cr] + 3,3 \times [\%Mo] + 20 \times [\%C] + 20 \times [\%N] - 0,5 \times [\%Mn] - 0,25 [\%Ni]$$

Die Menge an Molybdän in den erfindungsgemäßen Legierungen sollte daher zwischen 3,0 Gew.-% und 4,00 Gew.-% liegen.

**Beispiel 7: Untersuchung des Einflusses von Stickstoff auf eine erfindungsgemäße Legierung**

Zur Untersuchung des Einflusses von Stickstoff auf die mechanischen und chemischen Eigenschaften einer erfindungsgemäßen Legierung wurden gemäß Beispiel 1 die Legierungen A - L hergestellt, welche die folgenden Zusammensetzungen aufweisen:

|   | A | B | C | D | E | F | G | H | I | J | K | L |
|---|---|---|---|---|---|---|---|---|---|---|---|---|
| Cr | 16,0 | 16,0 | 16,0 | 16,0 | 16,0 | 16,0 | 16,0 | 16,0 | 16,0 | 16,0 | 16,0 | 16,0 |
| Mn | 11 | 11 | 11 | 11 | 11 | 11 | 11 | 11 | 11 | 11 | 11 | 11 |
| Mo | 3,19 | 3,19 | 3,19 | 3,19 | 3,19 | 3,19 | 3,19 | 3,19 | 3,19 | 3,19 | 3,19 | 3,19 |
| N | 0,36 | 0,41 | 0,45 | 0,49 | 0,52 | 0,55 | 0,58 | 0,61 | 0,65 | 0,70 | 0,75 | 0,80 |
| C | 0,15 | 0,15 | 0,15 | 0,15 | 0,15 | 0,15 | 0,15 | 0,15 | 0,15 | 0,15 | 0,15 | 0,15 |
| Ni | 0,03 | 0,03 | 0,03 | 0,03 | 0,03 | 0,03 | 0,03 | 0,03 | 0,03 | 0,03 | 0,03 | 0,03 |

(fortgesetzt)

|  | A | B | C | D | E | F | G | H | I | J | K | L |
|---|---|---|---|---|---|---|---|---|---|---|---|---|
| Si | 0,33 | 0,33 | 0,33 | 0,33 | 0,33 | 0,33 | 0,33 | 0,33 | 0,33 | 0,33 | 0,33 | 0,33 |
| P | 0,01 | 0,01 | 0,01 | 0,01 | 0,01 | 0,01 | 0,01 | 0,01 | 0,01 | 0,01 | 0,01 | 0,01 |

Die Legierungen A bis D weisen ein ferritisches Verhalten auf und sind daher als Material für Stents ungeeignet. Ihre Korrosionsbeständigkeiten, getestet wie unter Beispiel 4 beschrieben, sind äußerst gering. Die Legierung E weist ein deutlich besseres Korrosionsverhalten auf, das Durchbruchs- und Repassivierungspotential ist jedoch noch geringer als bei den Proben F bis I. Es liegt austenitisches Gefüge vor.

Die Legierungen F bis I weisen ein sehr gutes Korrosionsverhalten auf. Die chemische Beständigkeit ist deutlich höher als bei dem für vaskuläre Stents verwendeten Material 1.4441. Die Durchbruch- und Repassivierungspotentiale sind vergleichbar mit dem Material 2.4964 (L605).

Die Legierungen A bis H weisen eine mit dem Stickstoffgehalt steigende Dehngrenze von circa 450 MPa bis 600 MPa auf. Die Bruchdehnung der Proben A bis D erreichen circa 55 %. Die Bruchdehnung der Proben E bis J erreichen circa 65 %. Diese Parameter wurden wie unter Beispiel 5 beschrieben erhalten.

Die Legierungen I bis L weisen eine Dehngrenze von bis circa 640 MPa auf. Die Bruchdehnungen erreicht Werte von 55 % - 65 %. Die Legierung L weist eine höhere Korrosionsrate und ein geringeres Repassivierungspotential auf. Dies ist auf die Entstehung von Nitriden zurückzuführen, die bei höheren Stickstoffgehalten entstehen, und somit durch die um die Nitride entstehende Chrom- und Stickstoffverarmung die Korrosionsbeständigkeit verringern.

Die Abhängigkeit der Dehngrenze vom Stickstoffgehalt ist anhand folgender Formel ersichtlich:

$$\text{Dehngrenze (MPa)} = 251 + 33 \times \text{Mn (m\%)} + 313 \times [\text{N} + \text{C (m\%)}]$$

Da die Dehngrenze für die Anwendung als Stent unter 600 MPa liegen muss ist hinsichtlich der Dehngrenze ein möglichst geringer Stickstoffgehalt gefordert. In Kombination mit der Anforderung eines feinen Korns von möglichst G > 7 ist dies nur bis zu einem Stickstoffgehalt von maximal 0,7 % erzeugbar.

Die Menge an Stickstoff in den erfindungsgemäßen Legierungen sollte daher zwischen 0,55 Gew.-% und 0,7 Gew.-% betragen.

**Beispiel 8: Untersuchung des Einflusses von Kohlenstoff auf eine erfindungsgemäße Legierung**

Zur Untersuchung des Einflusses von Kohlenstoff auf die mechanischen und chemischen Eigenschaften einer erfindungsgemäßen Legierung wurden gemäß Beispiel 1 die Legierungen A - L hergestellt, welche die folgenden Zusammensetzungen aufweisen:

|  | A | B | C | D | E | F | G | H | I | J | K | L |
|---|---|---|---|---|---|---|---|---|---|---|---|---|
| Cr | 16,0 | 16,0 | 16,0 | 16,0 | 16,0 | 16,0 | 16,0 | 16,0 | 16,0 | 16,0 | 16,0 | 16,0 |
| Mn | 11 | 11 | 11 | 11 | 11 | 11 | 11 | 11 | 11 | 11 | 11 | 11 |
| Mo | 3,19 | 3,19 | 3,19 | 3,19 | 3,19 | 3,19 | 3,19 | 3,19 | 3,19 | 3,19 | 3,19 | 3,19 |
| N | 0,62 | 0,62 | 0,62 | 0,62 | 0,62 | 0,62 | 0,62 | 0,62 | 0,62 | 0,62 | 0,62 | 0,62 |
| C | 0,02 | 0,05 | 0,1 | 0,12 | 0,14 | 0,16 | 0,18 | 0,20 | 0,22 | 0,24 | 0,26 | 0,28 |
| Ni | 0,03 | 0,03 | 0,03 | 0,03 | 0,03 | 0,03 | 0,03 | 0,03 | 0,03 | 0,03 | 0,03 | 0,03 |
| Si | 0,33 | 0,33 | 0,33 | 0,33 | 0,33 | 0,33 | 0,33 | 0,33 | 0,33 | 0,33 | 0,33 | 0,33 |
| P | 0,01 | 0,01 | 0,01 | 0,01 | 0,01 | 0,01 | 0,01 | 0,01 | 0,01 | 0,01 | 0,01 | 0,01 |

Die Legierungen A - B weisen eine geringe Korrosionsbeständigkeit, gemessen wie unter Beispiel 4 beschrieben, auf. Insbesondere die Repassivierbarkeit ist im Vergleich zu den Legierungen D - H verringert. Die Legierungen weisen einen geringen Anteil an Delta-Ferrit auf. In der Legierung C ist Delta-Ferrit nur noch vereinzelt zu finden. Die Bruchdehnung beträgt circa 55 % - 60 % und die Dehngrenze circa 550 - 570 MPa.

Die Legierungen D - J weisen kein Delta-Ferrit auf. Die Legierung D weist eine höhere Korrosionsbeständigkeit als die Legierungen A - C auf. Die Repassivierbarkeit ist im Vergleich zu den Legierungen E - H verringert.

Die Legierungen E - H weisen eine sehr hohe Korrosionsbeständigkeit mit einem hohen Repassivierungspotential auf. Die Bruch- und Gleichmaßdehnung sind im Vergleich zu den anderen Legierungen erhöht. Die Bruchdehnung beträgt bis über 65 %. Die Dehngrenze beträgt circa 570 MPa - 600 MPa.

Die Legierung I weist eine hohe Korrosionsbeständigkeit auf. Vor allem das Repassivierungspotential ist vergleichend zu den Legierungen E - H verringert. Dies ist auf die Entstehung von vereinzelten Chromcarbiden zurückzuführen.

Die Legierung J weist eine deutlich verringerte Korrosionsbeständigkeit auf, was durch die Entstehung von Chromcarbiden zu erklären ist. Die Dehngrenzen der Legierungen I - L liegen bei circa 620 - 640 MPa. Noch weiter verringert ist die Korrosionsbeständigkeit der Legierungen K und L.

Die Menge an Kohlenstoff in den erfindungsgemäßen Legierungen sollte daher zwischen 0,10 Gew.-% und 0,20 Gew.-% liegen.

**Beispiel 9: Untersuchung des Einflusses von Kohlenstoff und Stickstoff auf eine erfindungsgemäße Legierung**

Zur Untersuchung des Einflusses von Kohlenstoff in Abhängigkeit des Stickstoffgehalts auf die mechanischen Eigenschaften einer erfindungsgemäßen Legierung wurden gemäß Beispiel 1 die Legierungen A - I1 hergestellt, welche die folgenden Zusammensetzungen aufweisen:

|     | A | B | C | D | E | F | G | H | I | J | K | L |
|-----|------|------|------|------|------|------|------|------|------|------|------|------|
| Cr  | 16,0 | 16,0 | 16,0 | 16,0 | 16,0 | 16,0 | 16,0 | 16,0 | 16,0 | 16,0 | 16,0 | 16,0 |
| Mn  | 11 | 11 | 11 | 11 | 11 | 11 | 11 | 11 | 11 | 11 | 11 | 11 |
| Mo  | 3,19 | 3,19 | 3,19 | 3,19 | 3,19 | 3,19 | 3,19 | 3,19 | 3,19 | 3,19 | 3,19 | 3,19 |
| N   | 0,50 | 0,6 | 0,7 | 0,8 | 0,9 | 0,50 | 0,6 | 0,7 | 0,8 | 0,9 | 0,50 | 0,6 |
| C   | 0,08 | 0,08 | 0,08 | 0,08 | 0,08 | 0,12 | 0,12 | 0,12 | 0,12 | 0,12 | 0,16 | 0,16 |
| Ni  | 0,03 | 0,03 | 0,03 | 0,03 | 0,03 | 0,03 | 0,03 | 0,03 | 0,03 | 0,03 | 0,03 | 0,03 |
| Si  | 0,33 | 0,33 | 0,33 | 0,33 | 0,33 | 0,33 | 0,33 | 0,33 | 0,33 | 0,33 | 0,33 | 0,33 |
| P   | 0,01 | 0,01 | 0,01 | 0,01 | 0,01 | 0,01 | 0,01 | 0,01 | 0,01 | 0,01 | 0,01 | 0,01 |

|     | M | N | O | P | Q | R | S | T | U | V | W |
|-----|------|------|------|------|------|------|------|------|------|------|------|
| Cr  | 16,0 | 16,0 | 16,0 | 16,0 | 16,0 | 16,0 | 16,0 | 16,0 | 16,0 | 16,0 | 16,0 |
| Mn  | 11 | 11 | 11 | 11 | 11 | 11 | 11 | 11 | 11 | 11 | 11 |
| Mo  | 3,19 | 3,19 | 3,19 | 3,19 | 3,19 | 3,19 | 3,19 | 3,19 | 3,19 | 3,19 | 3,19 |
| N   | 0,7 | 0,8 | 0,9 | 0,50 | 0,6 | 0,7 | 0,8 | 0,50 | 0,6 | 0,7 | 0,8 |
| C   | 0,16 | 0,16 | 0,16 | 0,20 | 0,20 | 0,20 | 0,20 | 0,24 | 0,24 | 0,24 | 0,24 |
| Ni  | 0,03 | 0,03 | 0,03 | 0,03 | 0,03 | 0,03 | 0,03 | 0,03 | 0,03 | 0,03 | 0,03 |
| Si  | 0,33 | 0,33 | 0,33 | 0,33 | 0,33 | 0,33 | 0,33 | 0,33 | 0,33 | 0,33 | 0,33 |
| P   | 0,01 | 0,01 | 0,01 | 0,01 | 0,01 | 0,01 | 0,01 | 0,01 | 0,01 | 0,01 | 0,01 |

|     | X    | Y    | Z    | A1   | B1   | C1   | D1   | E1   | F1   | G1   | H1   |
|-----|------|------|------|------|------|------|------|------|------|------|------|
| Cr  | 16,0 | 16,0 | 16,0 | 16,0 | 16,0 | 16,0 | 16,0 | 16,0 | 16,0 | 16,0 | 16,0 |
| Mn  | 11   | 11   | 11   | 11   | 11   | 11   | 11   | 11   | 11   | 11   | 11   |
| Mo  | 3,19 | 3,19 | 3,19 | 3,19 | 3,19 | 3,19 | 3,19 | 3,19 | 3,19 | 3,19 | 3,19 |
| N   | 0,50 | 0,6  | 0,7  | 0,8  | 0,50 | 0,6  | 0,7  | 0,8  | 0,5  | 0,6  | 0,7  |
| C   | 0,30 | 0,30 | 0,30 | 0,30 | 0,36 | 0,36 | 0,36 | 0,36 | 0,40 | 0,40 | 0,40 |
| Ni  | 0,03 | 0,03 | 0,03 | 0,03 | 0,03 | 0,03 | 0,03 | 0,03 | 0,03 | 0,03 | 0,03 |
| Si  | 0,33 | 0,33 | 0,33 | 0,33 | 0,33 | 0,33 | 0,33 | 0,33 | 0,33 | 0,33 | 0,33 |
| P   | 0,01 | 0,01 | 0,01 | 0,01 | 0,01 | 0,01 | 0,01 | 0,01 | 0,01 | 0,01 | 0,01 |

|     | I1   |
|-----|------|
| Cr  | 16,0 |
| Mn  | 11   |
| Mo  | 3,19 |
| N   | 0,80 |
| C   | 0,40 |
| Ni  | 0,03 |
| Si  | 0,33 |
| P   | 0,01 |

Die Legierungen A - B weisen eine geringe Korrosionsbeständigkeit auf. Insbesondere ist das Repassivierungspotential verringert. Die Bruchdehnung beträgt circa 55 % - 60 % und die Dehngrenze circa 530 - 560 MPa. Die Legierung C weist eine ausreichende chemische Beständigkeit mit leicht verringertem Repassivierungspotential auf. Die Bruchdehnung beträgt circa 60 % und die Dehngrenze circa 600 MPa. Die Legierungen D - E weisen eine gute chemische Beständigkeit auf. Die Bruchdehnung beträgt circa 55 % und die Dehngrenze circa 620 - 650 MPa.

Die Legierung F weist ein gutes Korrosionsverhalten mit einem hohen Repassivierungspotential auf. Insgesamt ist die chemische Beständigkeit den Legierungen G - H leicht unterlegen. Die Bruchdehnung beträgt circa 60 % und die Dehngrenze circa 550 MPa. Die Legierungen G - H weisen eine sehr gute chemische Beständigkeit auf. Die Bruchdehnung beträgt circa 60 % - 65 % und die Dehngrenze circa 580 - 600 MPa. Die Legierungen I - J weisen eine verringerte chemische Beständigkeit auf. Die Bruchdehnung beträgt circa 55 % - 60 % und die Dehngrenze circa 620 - 660 MPa.

Die Legierung K weist eine gute chemische Beständigkeit mit einem hohen Repassivierungspotential auf. Insgesamt ist die chemische Beständigkeit den Legierungen L - M leicht unterlegen. Die Bruchdehnung beträgt circa 60 % und die Dehngrenze circa 560 MPa. Die Legierungen L - M weisen eine sehr gute chemische Beständigkeit mit einem hohen Repassivierungspotential auf. Die Bruchdehnung beträgt circa 65 % und die Dehngrenze circa 590 - 610 MPa. Die Legierung N weist eine verringerte chemische Beständigkeit mit einem verringerten Repassivierungspotential auf. Die Bruchdehnung beträgt circa 55 % und die Dehngrenze circa 640 MPa.

Die Legierung O weist eine deutlich verringerte chemische Beständigkeit mit einem niedrigen Repassivierungspotential auf. Die Bruchdehnung beträgt circa 50 % und die Dehngrenze circa 660 MPa.

Die Legierungen P - Q weisen eine ausreichende chemische Beständigkeit mit einem hohen Repassivierungspotential auf. Die Bruchdehnung beträgt circa 60 % und die Dehngrenze circa 570 - 600 MPa. Die Legierung R weist eine sehr gute chemische Beständigkeit mit einem hohen Repassivierungspotential auf. Die Bruchdehnung beträgt circa 65 % und die Dehngrenze circa 630 MPa. Die Legierung S weist eine höhere Korrosionsrate mit einem geringen Repassivierungspotential auf. Die Bruchdehnung beträgt circa 60 % und die Dehngrenze circa 670 MPa.

Die Legierung T - U weisen eine noch ausreichende chemische Beständigkeit mit einem ausreichenden Repassivierungspotential auf. Die Bruchdehnung beträgt circa 60 % und die Dehngrenze circa 590 - 620 MPa. Die Legierungen V - W weisen eine verringerte chemische Beständigkeit mit einem verringerten Repassivierungspotential auf. Die Bruchdehnung beträgt circa 55 % und die Dehngrenze circa 660 - 690 MPa.

Die Legierungen X - Y haben eine im Vergleich zur Legierung G verringerte chemische Beständigkeit. Auch das Repassivierungspotential ist verringert. Die Bruchdehnung erreicht Werte von etwa 55 %. Die Dehngrenze beträgt 610 - 640 MPa. Die Legierungen Z - A1 weisen eine noch weiter verringerte chemische Beständigkeit auf. Die Bruchdehnung beträgt circa 50 %. Die Dehngrenze erreicht Werte von 670 - 700 MPa. Die polierten Oberflächen zeigen vermehrt Vertiefungen auf.

Die Legierungen B1 - C1 weisen eine deutlich verringerte chemische Beständigkeit auch hinsichtlich des Repassivierungspotentials auf. Die Bruchdehnung erreicht Werte von 50 - 55 %. Die Dehngrenze liegt bei 620 - 650 MPa. Die polierten Oberflächen zeigen vermehrt Vertiefungen, was auf feine Ausscheidungserscheinungen hinweist.

Die Legierungen D1 - E1 weisen eine geringe chemische Beständigkeit auf und sind daher nicht als Stentwerkstoff zu verwenden. Die Bruchdehnung beträgt 45 - 50 %. Die Dehngrenze erreicht Werte von 680 - 710 MPa. Die polierten Oberflächen vor allem von Legierung E1 weisen vermehrt größere Vertiefungen auf. Es treten Ausscheidungseffekte auf.

Die Legierungen F1 - G1 weisen eine stark verringerte chemische Beständigkeit auf und sind daher nicht als Stentwerkstoff verwendbar. Die Bruchdehnung beträgt 45 - 50 %. Die Dehngrenze erreicht Werte von 640 - 670 MPa. Im polierten Zustand sind vermehrt Vertiefungen aber auch Erhöhungen vorhanden, die eine Folge von Ausscheidungen sind.

Die Legierungen H1 - 11 sind chemisch nicht beständig. Die Legierungen weisen ein geringes Durchbruch- und Repassivierungspotential auf. Die Bruchdehnung beträgt etwa 40 %. Die Dehngrenze erreicht Werte von 690 - 720 MPa. Die Legierungen H1 - I1 weisen nach der Politur keine polierten Oberflächen auf, die in der Anwendung als Stent geeignet sind.

Die Legierungen G und H so wie L und M als auch R zeigen durch die Vereinigung von positiven Eigenschaften eine besondere Eignung als Stentmaterial. Sie weisen alle einen Stickstoffgehalt zwischen 0,6 % und 0,7 % auf, einen Kohlenstoffgehalt zwischen 0,12 % und 0,2 % und ein Verhältnis von N : C von 3,50 bis 5,83 auf.

**Patentansprüche**

1. Legierung die bezogen auf das Gesamtgewicht der Legierung folgende Bestandteile enthält:

| | | |
|---|---|---|
| 14,0 Gew.-% - | 16,5 ,Gew.-%, | Chrom |
| 10,0 Gew.-% - | 12,0 Gew.-% | Mangan |
| 3,0 Gew.-% - | 4,0 Gew.-% | Molybdän |
| 0,55 Gew.-% - | 0,70 Gew.-% | Stickstoff |
| 0,10 Gew.-% - | 0,20 Gew.-% | Kohlenstoff |
| 0,00 Gew.-% - | 0,05 Gew.-% | Nickel |
| 0,00 Gew.-% - | 1,00 Gew.-% | Silizium |
| 0,00 Gew.-% - | 0,1 Gew.-% | Phosphor und Schwefel |

der Rest bis auf 100 Gew.-% ist Eisen, und unvermeidbare Verunreinigungen.

2. Legierung gemäß Anspruch 1 deren Korngröße G zwischen 6 und 10 liegt.

3. Verwendung der Legierung gemäß einem der Ansprüche 1 oder 2 als Werkstoff zur Herstellung eines Stents.

4. Stent bestehend aus einer Stahllegierung gemäß einem der Ansprüche 1 - 3.

5. Stent nach Anspruch 4, wobei es sich bei dem Stent um einen Stent für Blutgefäße, Harnwege, Atemwege, Gallenwege oder den Verdauungstrakt handelt.

**Claims**

1. Alloy containing the following components based on the total weight of the alloy:

| | | |
|---|---|---|
| 14.0 % by wt. - | 16.5 % by wt. | chromium |
| 10.0 % by wt. - | 12.0 % by wt. | manganese |
| 3.0 % by wt. - | 4.0 % by wt. | molybdenum |
| 0.55 % by wt. - | 0.70 % by wt. | nitrogen |
| 0.10 % by wt. - | 0.20 % by wt. | carbon |
| 0.00 % by wt. - | 0.05 % by wt. | nickel |
| 0.00 % by wt. - | 1.00 % by wt. | silicon |

(continued)

| 0.00 % by wt. - | 0.1 % by wt. | phosphorus and sulfur |

the rest up to 100 % by wt. is iron and unavoidable impurities.

2. Alloy according to claim 1 having a grain size G between 6 and 10.

3. Use of the alloy according to claim 1 or 2 as material for the production of a stent.

4. Stent consisting of a steel alloy according to any one of claims 1 - 3.

5. Stent according to claim 4, wherein the stent is a stent for blood vessels, urinary tracts, respiratory tracts, biliary tracts or the digestive tract.


**Revendications**

1. Un alliage contenat par rapport au poids total de l'alliage les composants suivants:

| 14,0% par poids - | 16,5% par poids | chrome |
| 10,0% par poids - | 12,0% par poids | manganèse |
| 3,0% par poids - | 4,0% par poids | molybdène |
| 0,55% par poids - | 0,70% par poids | azote |
| 0,10% par poids - | 0,20% par poids | carbone |
| 0,00% par poids - | 0,05% par poids | nickel |
| 0,00% par poids - | 1,00% par poids | silicium |
| 0,00% par poids - | 0,1 % par poids | phosphore et souffre |

le reste jusqu'à 100,0 % par poids fer et impuretés inévitables.

2. L'alliage selon la revendication 1 ayant une taille de la grain G de 6 à 10.

3. Utilisation de l'alliage selon la revendicatuin 1 ou 2 en tant que matériau pour la fabrication d'un stent.

4. Un stent constitué par d'un alliage d'acier selon une des revendications 1 - 3.

5. Le stent selon la revendication 4, où le stent est un stent pour les vaisseaux sanguins, les voies urinaires, le voies respiratoires, les voies biliaires ou la voie digestive.

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- US 6508832 B
- EP 640695 A1
- EP 1087029 A2
- EP 0875591 B1
- DE 19513407 C1
- EP 1025273 B1
- EP 1579886 A1

**In der Beschreibung aufgeführte Nicht-Patentliteratur**

- **CHEN et al.** *Computational Materials Science,* 2009, 572-578